# EUROPEAN PATENT SPECIFICATION

(11) **EP 1 368 054 B1**
(45) Date of publication and mention of the grant of the patent: **03.10.2007**
(21) Application number: 02733095.0
(22) Date of filing: 08.03.2002
(51) Int. Cl.: A61K 38/17, A61P 35/00

(54) **USING NEURAL THREAD PROTEINS TO TREAT TUMORS**
VERWENDUNG VON NEUROFILAMENTPROTEINEN ZUR BEHANDLUNG VON TUMOREN
METHODES D'UTILISATION DES PROTEINES NTP POUR TRAITER DES TUMEURS

(30) Priority: 08.03.2001 US 273957 P
(43) Date of publication of application: 10.12.2003
(60) Divisional application: 07110879.9
(73) Proprietor: Nymox Pharmaceutical Corporation, St. Laurent, Quebec H4M 2V2 (CA)
(72) Inventor: AVERBACK, Paul, Quebec H4W 4J1 (CA)
(74) Representative: Schouboe, Anne
(86) International application number: PCT/IB2002/001959
(87) International publication number: WO 2002/074323

(56) References cited:
- WO-A-01/46237
- WO-A-01/46440
- WO-A-96/15272

### SEQUENCE LISTING

<110> NYMOX PHARMACEUTICAL CORPORATION
<120> METHODS OF USING NEURAL THREAD PROTEINS TO TREAT TUMORS AND CONDITIONS REQUIRING THE REMOVAL OR DESTRUCTION OF CELLS
<130> 018792-0198
<140> PCT/US02/05103
   <141> 2002-03-08
<150> 60/273,957
   <151> 2001-03-08
<160> 11
<170> PatentIn Ver. 2.1
<210> 1
   <211> 1442
   <212> DNA
   <213> Homo sapiens
<220>
   <221> CDS
   <222> (15)..(1139)
<400> 1
   tttttttttt tgag atg gag ttt tcg ctc ttg ttg ccc agg ctg gag tgc    50
   aat ggc gca atc tca gct cac cgc aac ctc cgc ctc ccg ggt tca agc    98
   gat tct cct gcc tca gcc tcc cca gta gct ggg att aca ggc atg tgc    146
   acc cac gct cgg cta att ttg tat ttt ttt tta gta gag atg gag ttt    194
   ctc cat gtt ggt cag gct ggt ctc gaa ctc ccg acc tca gat gat ccc    242
   tcc gtc tcg gcc tcc caa agt gct aga tac agg act ggc cac cat gcc    290
   cgg ctc tgc ctg gct aat ttt tgt ggt aga aac agg gtt tca ctg atg    338
   tgc cca agc tgg tct cct gag ctc aag cag tcc acc tgc ctc agc ctc     386
   cca aag tgc tgg gat tac agg cgt gca gcc gtg cct ggc ctt ttt att     434
   tta ttt ttt tta aga cac agg tgt ccc act ctt acc cag gat gaa gtg     482
   cag tgg tgt gat cac agc tca ctg cag cct tca act cct gag atc aag     530
   cat cct cct gcc tca gcc tcc caa gta gct ggg acc aaa gac atg cac     578
   cac tac acc tgg cta att ttt att ttt att ttt aat ttt ttg aga cag     626
   agt ctc aac tct gtc acc cag gct gga gtg cag tgg cgc aat ctt ggc     674
   tca ctg caa cct ctg cct ccc ggg ttc aag tta ttc tcc tgc ccc agc     722
   ctc ctg agt agc tgg gac tac agg cgc cca cca cgc cta gct aat ttt     770
   ttt gta ttt tta gta gag atg ggg ttc acc atg ttc gcc agg ttg atc     818
   ttg atc tct gga cct tgt gat ctg cct gcc tcg gcc tcc caa agt gct     866
   ggg att aca ggc gtg agc cac cac gcc cgg ctt att ttt aat ttt tgt     914
   ttg ttt gaa atg gaa tct cac tct gtt acc cag gct gga gtg caa tgg     962
   cca aat ctc ggc tca ctg caa cct ctg cct ccc ggg ctc aag cga ttc     1010
   tcc tgt ctc agc ctc cca agc agc tgg gat tac ggg cac ctg cca cca   1058
   cac ccc gct aat ttt tgt att ttc att aga ggc ggg gtt tca cca tat   1106
   ttg tca ggc tgg tct caa act cct gac ctc agg tgacccacct gcctcagcct 1159
   tccaaagtgc tgggattaca ggcgtgagcc acctcaccca gccggctaat ttagataaaa 1219
   aaatatgtag caatgggggg tcttgctatg ttgcccaggc tggtctcaaa cttctggctt 1279
   catgcaatcc ttccaaatga gccacaacac ccagccagtc acatttttta aacagttaca 1339
   tctttatttt agtatactag aaagtaatac aataaacatg tcaaacctgc aaattcagta 1399
   gtaacagagt tcttttataa cttttaaaca aagctttaga gca                   1442
<210> 2
   <211> 122
   <212> PRT
   <213> Unknown Organism
<220>
   <223> Description of Unknown Organism: Neural thread protein
<400> 2
   Met Met Val Cys Trp Asn Arg Phe Gly Lys Trp Val Tyr Phe Ile Ser
   1               5                   10                  15
   Ala Ile Phe Asn Phe Gly Pro Arg Tyr Leu Tyr His Gly Val Pro Phe
               20                  25                  30
   Tyr Phe Leu Ile Leu Val Arg Ile Ile Ser Phe Leu Ile Gly Asp Met
           35                  40                  45
   Glu Asp Val Leu Leu Asn Cys Thr Leu Leu Lys Arg Ser Ser Arg Phe
       50                  55                  60
   Arg Phe Trp Gly Ala Leu Val Cys Ser Met Asp Ser Cys Arg Phe Ser
   65                  70                  75                  80
   Arg Val Ala Val Thr Tyr Arg Phe Ile Thr Leu Leu Asn Ile Pro Ser
                   85                  90                  95
   Pro Ala Val Trp Met Ala Arg Asn Thr Ile Asp Gln Gln Val Leu Ser
               100                 105                 110
   Arg Ile Lys Leu Glu Ile Lys Arg Cys Leu
           115                 120
<210> 3
   <211> 112
   <212> PRT
   <213> Homo sapiens
<400> 3
<210> 4
   <211> 106
   <212> PRT
   <213> Homo sapiens
<400> 4
   Met Trp Thr Leu Lys Ser Ser Leu Val Leu Leu Leu Cys Leu Thr Cys
   1               5                   10                  15
   Ser Tyr Ala Phe Met Phe Ser Ser Leu Arg Gln Lys Thr Ser Glu Pro
               20                  25                  30
   Gln Gly Lys Val Pro Cys Gly Glu His Phe Arg Ile Arg Gln Asn Leu
           35                  40                  45
   Pro Glu His Thr Gln Gly Trp Leu Gly Ser Lys Trp Leu Trp Leu Leu
       50                  55                  60
   Phe Ala Val Val Pro Phe Val Ile Leu Lys Cys Gln Arg Asp Ser Glu
   65                  70                  75                  80
   Lys Asn Lys Val Arg Met Ala Pro Phe Phe Leu His His Ile Asp Ser
                   85                  90                  95
   Ile Ser Gly Val Ser Gly Lys Arg Met Phe
               100                 105
<210> 5
   <211> 106
   <212> PRT
   <213> Homo sapiens
<400> 5
   Met Phe Phe Val Leu Tyr Arg Phe Cys Phe Cys Phe Phe Glu Thr Glu
   1               5                   10                  15
   Ser His Ser Leu Thr Gln Ala Gly Val Gln Trp Cys Glu Leu Gly Ser
               20                  25                  30
   Pro Gln Pro Leu Pro Ser Gly Phe Lys Arg Phe Ser Cys Leu Ser Leu
           35                  40                  45
   Leu Ser Ser Trp Asp Tyr Ser His Glu Pro Pro His Pro Val Ile Cys
       50                  55                  60
   Ser Phe Leu Met Glu Lys Cys Leu Ile Leu Tyr Lys Pro Asn Gly Asp
   65                  70                  75                  80
   Thr Ile Gly Pro Ile Leu Val Gln Gln Gly Lys Arg Gln Lys Leu Tyr
                   85                  90                  95
   Ile Ser Ala Asp Leu Val His Leu Ile Ala
               100                 105
<210> 6
   <211> 98
   <212> PRT
   <213> Unknown Organism
<220>
   <223> Description of Unknown Organism: Neural thread protein
<400> 6
   Glu Ala Tyr Tyr Thr Met Leu His Leu Pro Thr Thr Asn Arg Pro Lys
   1               5                   10                  15
   Ile Ala His Cys Ile Leu Phe Asn Gln Pro His Ser Pro Arg Ser Asn
               20                  25                  30
   Ser His Ser His Pro Asn Pro Leu Lys Leu His Arg Arg Ser His Ser
           35                  40                  45
   His Asn Arg Pro Arg Ala Tyr Ile Leu Ile Thr Ile Leu Pro Ser Lys
       50                  55                  60
   Leu Lys Leu Arg Thr His Ser Gln Ser His His Asn Pro Leu Ser Arg
   65                  70                  75                  80
   Thr Ser Asn Ser Thr Pro Thr Asn Ser Phe Leu Met Thr Ser Ser Lys
                   85                  90                  95
   Pro Arg
<210> 7
   <211> 75
   <212> PRT
   <213> Unknown Organism
<220>
   <223> Description of Unknown Organism: Neural thread protein
<400> 7
   Ser Ser Ser Leu Gly Leu Pro Lys Cys Trp Asp Tyr Arg His Glu Leu
   1 5 10 15
   Leu Ser Leu Ala Leu Met Ile Asn Phe Arg Val Met Ala Cys Thr Phe
   20 25 30
   Lys Gln His Ile Glu Leu Arg Gln Lys Ile Ser Ile Val Pro Arg Lys
   35 40 45
   Leu Cys Cys Met Gly Pro Val Cys Pro Val Lys Ile Ala Leu Leu Thr
   50 55 60
   Ile Asn Gly His Cys Thr Trp Leu Pro Ala Ser
   65 70 75
<210> 8
   <211> 68
   <212> PRT
   <213> Unknown Organism
<220>
   <223> Description of.Unknown Organism: Neural thread protein
<400> 8
   Met Phe Val Phe Cys Leu Ile Leu Asn Arg Glu Lys Ile Lys Gly Gly
   1 5 10 15
   Asn Ser Ser Phe Phe Leu Leu Ser Phe Phe Phe Ser Phe Gln Asn Cys
   20 25 30
   Cys Gln Cys Phe Gln Cys Arg Thr Thr Glu Gly Tyr Ala Val Glu Cys
   35 40 45
   Phe Tyr Cys Leu Val Asp Lys Ala Ala Phe Glu Cys Trp Trp Phe Tyr
   50 55 60
   Ser Phe Asp Thr
   65
<210> 9
   <211> 61
   <212> PRT
   <213> Homo sapiens
<400> 9
   Met Glu Pro His Thr Val Ala Gln Ala Gly Val Pro Gln His Asp Leu
   1 5 10 15
   Gly Ser Leu Gln Ser Leu Leu Pro Arg Phe Lys Arg Phe Ser Cys Leu
   20 25 30
   Ile Leu Pro Lys Ile Trp Asp Tyr Arg Asn Met Asn Thr Ala Leu Ile
   35 40 45
   Lys Arg Asn Arg Tyr Thr Pro Glu Thr Gly Arg Lys Ser
   50 55 60
<210> 10
   <211> 375
   <212> PRT
   <213> Homo sapiens
<400> 10
<210> 11
   <211> 6
   <212> PRT
   <213> Artificial Sequence
<220>
   <223> Description of Artificial Sequence: 6-His tag
<400> 11
   His His His His His His
   1               5

## Description

### FIELD OF THE INVENTION

The present invention is directed use of a therapeutically effective amount of a neural thread protein for the manufacture of a composition for treating tumors. The composition can be administered intramuscularly, orally, intravenously, intrathecally, intratumorally, intranasally, topically, transdermally, etc., either alone or conjugated to a carrier.

### BACKGROUND OF THE INVENTION

The essence of many medical treatments and procedures involves the removal or destruction of harmful or unwanted tissue. Examples of such important treatments include the surgical removal of cancerous growths, the destruction of metatastic tumors through chemotherapy, and the reduction of glandular (e.g. prostate) hyperplasia. Other examples include the removal of unwanted facial hair, the removal of warts, and the removal of unwanted fatty tissue.

There is an obvious need for an effective agent that will destroy and hence either facilitate the removal of or inhibit the further growth of harmful or unwanted tumor cells but will have mainly local effects and minimal or absent systemic toxicity.

Neural thread proteins and their related molecules are such agents.

### Cancer and Benign Overgrowths

Cancer is an abnormality in a cell's internal regulatory mechanisms that results in uncontrolled growth and reproduction of the cell. Normal cells make up tissues, and when these cells lose their ability to behave as a specified, controlled, and coordinated unit (dedifferentiation) the defect leads to disarray amongst the cell population. When this occurs, a tumor is formed.

Benign overgrowths of tissue are abnormalities in which it is desirable to remove cells from an organism. Benign tumors are cellular proliferations that do not metastasize throughout the body but do, however, cause disease symptoms. Such tumors can be lethal if they are located in inaccessible areas in organs such as the brain. There are benign tumors of organs including lung, brain, skin, pituitary, thyroid, adrenal cortex and medulla, ovary, uterus, testis, connective tissue, muscle, intestines, ear, nose, throat, tonsils, mouth, liver, gall bladder, pancreas, prostate, heart, and other organs.

### Cancer Treatments

Surgery is often the first step in the treatment of cancer. The objective of surgery varies. Sometimes it is used to remove as much of the evident tumor as possible, or at least to "debulk" it (remove the major bulk(s) of tumor so that there is less that needs to be treated by other means). Depending on the cancer type and location, surgery may also provide some symptomatic relief to the patient. For instance, if a surgeon can remove a large portion of an expanding brain tumor, the pressure inside the skull will decrease, leading to improvement in the patient's symptoms.

Not all tumors are amenable to surgery. Some may be located in parts of the body that make them impossible to completely remove. Examples of these would be tumors in the brainstem (a part of the brain that controls breathing) or a tumor which has grown in and around a major blood vessel. In these cases, the role of surgery is limited due to the high risk associated with tumor removal.

In some cases, surgery is not used to debulk tumor because it is simply not necessary. An example is Hodgkin's lymphoma, a cancer of the lymph nodes that responds very well to combinations of chemotherapy and radiation therapy. In Hodgkin's lymphoma, surgery is rarely needed to achieve cure, but almost always used to establish a diagnosis.

Chemotherapy is another common form of cancer treatment. Essentially, it involves the use of medications (usually given by mouth or injection) which specifically attack rapidly dividing cells (such as those found in a tumor) throughout the body. This makes chemotherapy useful in treating cancers that have already metastasized, as well as tumors that have a high chance of spreading through the blood and lymphatic systems but are not evident beyond the primary tumor. Chemotherapy may also be used to enhance the response of localized tumors to surgery and radiation therapy. This is the case, for example, for some cancers of the head and neck.

Unfortunately, other cells in the human body which also normally divide rapidly (such as the lining of the stomach and hair) are also affected by chemotherapy. For this reason, some (though not all) chemotherapy agents induce nausea or hair loss. These side effects are temporary, and doctors have medications they can provide to help alleviate many of these side effects. So, in general, chemotherapy treatments are well tolerated by patients. As our knowledge has continued to grow, researchers have devised newer chemotherapeutic agents that are not only better at killing cancer cells, but that also have fewer side effects for the patient.

Chemotherapy is administered to patients in a variety of ways. Some are pills that are taken daily or once a week or some other schedule and some are administered by an intravenous injection. For injectable chemotherapy, a patient goes to the doctor's office or hospital for treatment. Other chemotherapeutic agents require continuous infusion into the bloodstream, 24 hours a day. For these types of chemotherapy, a minor surgical procedure is performed to implant a small pump worn by the patient. The pump then slowly administers the medication. In many cases, a permanent port is placed in a patient's vein to eliminate the requirement of repeated needle sticks.

Radiation therapy is another commonly used weapon in the fight against cancer. Radiation kills cancer by damaging the DNA within the tumor cells. The radiation is "applied" two possible ways. The first, and most common, involves pointing a beam of radiation at the patient in a highly precise manner, focusing on the tumor. To do this, a patient lies on a table and the beam moves around him/her. This only takes a couple minutes, but is done five days per week for 3-6 weeks (depending on the type of tumor), to achieve a particular total prescribed "dose."

Another radiation method sometimes employed, called brachytherapy, involves taking radioactive pellets ("seeds") or wires and implanting them in the body in the area of the tumor. The implants can be temporary or permanent. For permanent implants, the radiation in the seeds "decays" or fades away over a period of days or weeks so that the patient is not radioactive. For temporary implants, the entire dose of radiation is usually delivered in about two days, and the patient must remain in the hospital during that time. For both types of brachytherapy, radiation is generally delivered to a very targeted area to gain local control over a cancer (as opposed to treating the whole body, as chemotherapy does.)

Some highly selected patients may be referred for bone marrow transplants. This procedure is usually performed either because a patient has a cancer that is particularly aggressive or because they have a cancer that has relapsed after being treated with conventional therapy. Bone marrow transplantation is a complicated procedure. There are many types, and they vary in their potential for causing side effects and cure. Most transplants are performed at special centers, and in many cases their use is considered investigational.

There are a number of other therapies, though most of them are still being explored in clinical trials and have not yet become standard care. Examples include the use of immunotherapy, monoclonal antibodies, anti-angiogenesis factors, and gene therapy.

Immunotherapy: There are various techniques designed to help the patient's own immune system fight the cancer, quite separately from radiation or chemotherapy. Oftentimes, to achieve the goal researchers inject the patient with a specially derived vaccine. Most research in this area has been conducted on melanoma, though other cancers are also now being targeted.

Monoclonal Antibodies: These are small proteins that are especially designed to attach to cancerous cells (and not normal cells) by taking advantage of differences between cancerous and non-cancerous cells in their cell membranes. Before administering the antibodies to the patient, they are often "tagged" (attached) to various cytotoxic compounds or are made radioactive, such that the antibody preferentially targets the cancerous cells, thereby delivering the toxic agent to the desired cells.

Anti-Angiogenesis Factors: As cancer cells rapidly divide and tumors grow, they can soon outgrow their blood supply. To compensate for this, some tumors secrete a compound which has been shown to help induce the growth of blood vessels in their vicinity, thus assuring the cancer cells a continuous supply of nutrients. Recently, researchers have been studying ways to turn this process off (stopping the growth of blood vessels).

Gene Therapy: Cancer is the product of a series of mutations that ultimately lead to the production of a cancer cell and its excessive proliferation. Cancers can be treated by introducing genes to the cancer cells that will act either to check or stop the cancer's proliferation, turn on the cell's programmed cell mechanisms to destroy the cell, enhance immune recognition of the cell, or express a pro-drug that converts to a toxic metabolite or a cytokine that inhibits tumor growth.

### Treatment of Benign Tumors

Benign tumors can also be treated by a variety of methods including surgery, radiotherapy, drug therapy, thermal or electric ablation, cryotherapy, and others. Although benign tumors do not metastasize, they can grow large and they can recur. Surgical extirpation of benign tumors has all the difficulties and side effects of surgery in general and oftentimes must be repeatedly performed for some benign tumors, such as for pituitary adenomas, meningeomas of the brain, prostatic hyperplasia, and others.

### Neural Thread Proteins

Neural thread proteins (NTP) are a novel family of recently characterized brain proteins. One member of this family, AD7C-NTP, is a ∼41 kD membrane associated phosphoprotein with functions related to neuritic sprouting and cell death (de la Monte et al., J. Clin. Invest., 100:3093-3104 (1997); de la Monte et al., Alz.. Rep., 2:327-332 (1999); de la Monte SM and Wands JR, Journal of Alzheimer's Disease, 3:345-353 (2001)). The gene and predicted protein sequence for AD7C-NTP has been identified and described (de la Monte et al., J. Clin. Invest., 100:3093-3104 (1997)). In addition to the ∼41 kD species, other species of neural thread protein (∼26 kD, ∼21 kD, ∼17 kD, and ∼15 kD) have been identified and associated with neuroectodermal tumors, astrocytomas, and glioblastomas and with injury due to hypoxia, schema, or cerebral infarction (Xu et al., Cancer Research, 53:3823-3829; de la Monte et al., J. Neuropathol. Exp. Neurol., 55(10):1038-50 (1996), de la Monte et al., J. Neurol. Sci., 138(1-2):26-35 (1996); de la Monte et al., J. Neurol. Sci., 135(2):118-25 (1996); de la Monte et al., J. Clin. Invest., 100: 3093-3104 (1997); and de la Monte et al., Alz.. Rep., 2:327-332 (1999)).

Neural thread protein has been described and claimed in U.S. Patent Nos. 5,948,634; 5,948,888; and 5,830,670, all for "Neural Thread Protein Gene Expression and Detection of Alzheimer's Disease" and in U.S. Patent No. 6,071,705 for "Method of Detecting Neurological Disease or Dysfunction."

There is compelling evidence linking AD7C-NTP with Alzheimer's disease (AD). AD7C-NTP mRNA is upregulated in AD brain compared to controls; AD7C-NTP protein levels in brain and in CSF are higher in AD than controls; and AD7C-NTP immunoreactivity is found in senile plaques, in neurofibrillary tangles (NFT), in degenerating neurons, neuropil threads, and dystrophic neurotic sprouts in AD and Down syndrome brains (Ozturk et al., Proc. Natl. Acad. Sci. USA, 86:419.423 (1989); de la Monte et al., J. Clin. Invest., 86(3):1004-13 (1990); de la Monte et al., J. Neurol. Sci., 113(2):152-64 (1992); de la Monte et al., Ann. Neurol., 32(6):733-42 (1992); de la Monte et al., J. Neuropathol. Exp. Neurol., 55(10):1038-50 (1996), de la Monte et al., J. Neurol. Sci., 138(1-2):26-35 (1996); de la Monte et al., J. Neurol. Sci., 135(2):118-25 (1996); de la Monte et al., J. Clin. Invest., 100:3093-3104 (1997); and de la Monte et al., Alz.. Rep., 2:327-332 (1999)). AD7C-NTP has also been identified Down's Syndrome brain tissue (Wands *et al.,* International Patent Publication No. WO 90/06993; de la Monte et al., Alz.. Rep., 2:327-332 (1999)). AD7C-NTP has also been linked to the process of cell death in Alzheimer's disease (de la Monte and Wands, J. Neuropatho. and Exp. Neuro., 60:195-207 (2001)).

There remains a need in the art for new, less toxic treatments for treating tumors. The present invention satisfies these needs.

### SUMMARY OF THE INVENTION

The present invention is directed to use of a therapeutically effective amount of a neural thread protein for the manufacture of a composition for treating tumors. The composition may be administered to a mammal in need of a therapeutically effective amount of neural thread protein (NTP).

NTP can be administered alone or conjugated to a carrier or an antibody. In addition, NTP may be used in conjunction with other therapies for treating benign and malignant tumors.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE FIGURES

- Figure 1:: Shows the complete amino acid sequence and nucleic acid sequence of the AD7c-NTP gene and the AD7c-NTP protein product of that gene (Sequences 120 and 121 from U.S. Patent Nos. 5,830,670, 5,948,634, and 5,948,888; de la Monte et al., J. Clin. Invest., 100:3093-3104 (1997); NCBI Entrez-Protein Accession # AAC08737; PID g3002527) [SEQ ID NO. 1].
- Figure 2:: Shows the complete amino acid sequences of the 122 amino acid neural thread protein (Sequence 40 from U.S. Patent Nos. 5,830,670,5,948,634, and 5,948,888; NCBI Entrez-Protein Accession # AAE25447 PID g10048540) [SEQ ID NO. 2].
- Figure 3:: Shows the complete amino acid sequences of the 112 amino acid neural thread protein (NCBI Entrez-Protein Accession #XP_032307 PID g15928971) [SEQ ID NO. 3].
- Figure 4:: Shows the complete amino acid sequences of a 106 amino acid neural thread protein-like protein (NCBI Entrez-Protein Accession #AAH 14951 PID g15928971) [SEQ ID NO. 4].
- Figure 5:: Shows the complete amino acid sequences of a 106 amino acid neural thread protein-like protein (NCBI Entrez-Protein Accession #XP_039102 PID g18599339) [SEQ ID NO. 5].
- Figure 6:: Shows the complete amino acid sequences of the 98 amino acid neural thread protein (Sequence 30 from U.S. Patent Nos. 5,830,670, 5,948,634, and 5,948,888; NCBI Entrez-Protein Accession # AAE25445, PID g10048538) [SEQ ID NO. 6].
- Figure 7:: Shows the complete amino acid sequences of the 75 amino acid neural thread protein (Sequence 48 from U.S. Patent Nos. 5,830,670, 5,948,634, and 5,948,888; NCBI Entrez-Protein Accession #AAE25448, PID g10048541) [SEQ ID NO. 7].
- Figure 8:: Shows the complete amino acid sequences of the 68 amino acid neural thread protein (Sequence 36 from U.S. Patent Nos. 5,830,670, 5,948,634, and 5,948,888; NCBI Entrez-Protein Accession #AAE25446, PID g10048539) [SEQ ID NO. 8].
- Figure 9:: Shows the complete amino acid sequences of the 61 amino acid neural thread protein-like protein (NCBI Entrez-Protein Accession #AAH02534, PID g12803421) [SEQ ID NO. 9].
- Figure 10:: Shows subcutaneous tissue and muscle necrosis at AD7C-NTP injection site, 24 hours post-injection. Optical microscopy, hematoxylin and eosin stain, x400;
- Figure 11:: Shows subcutaneous tissue and muscle necrosis at AD7C-NTP injection site, 24 hours post-injection. Optical microscopy, hematoxylin and eosin stain, x400;

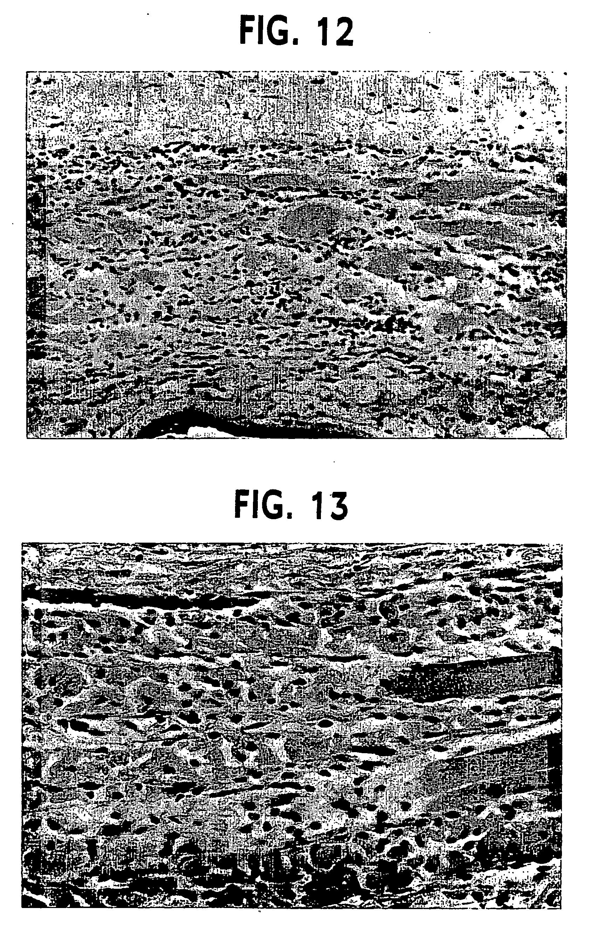

- Figure 12:: Shows subcutaneous tissue and muscle necrosis at AD7C-NTP injection site, 24 hours post-injection. Optical microscopy, hematoxylin and eosin stain, x400;
- Figure 13:: Shows subcutaneous tissue and muscle necrosis at AD7C-NTP injection site, 24 hours post-injection. Optical microscopy, hematoxylin and eosin stain, x1000;

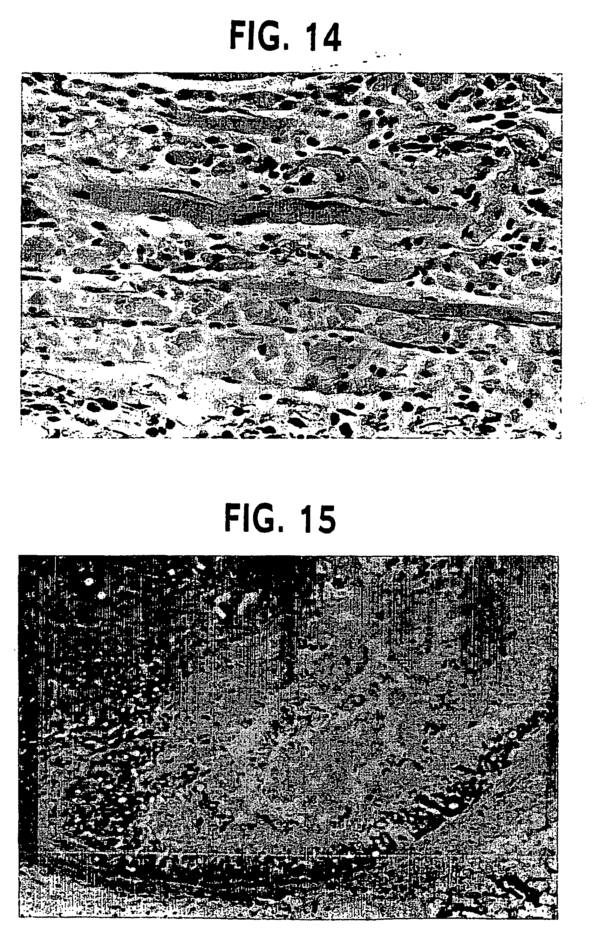

- Figure 14:: Shows subcutaneous tissue and muscle necrosis at AD7C-NTP injection site, 24 hours post-injection. Optical microscopy, hematoxylin and eosin stain, x1000;
- Figure 15:: Shows Tumor necrosis 24 hours after AD7C-NTP injection. Optical microscopy, hematoxylin and eosin stain x200;

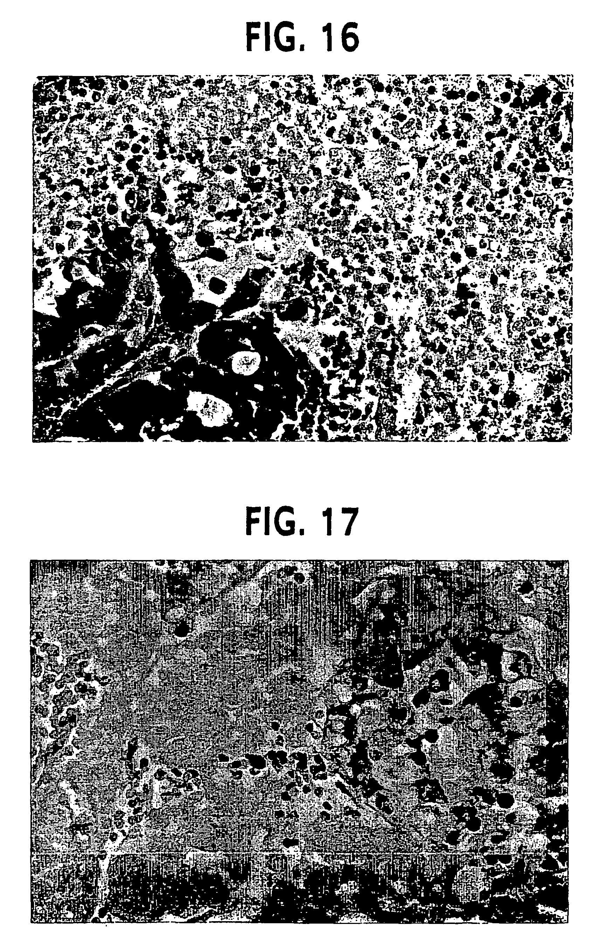

- Figure 16:: Shows tumor necrosis 24 hours after AD7C-NTP injection. Optical microscopy, hematoxylin and eosin stain x400;
- Figure 17:: Shows tumor necrosis 24 hours after AD7C-NTP injection. Optical microscopy, hematoxylin and eosin stain x1000

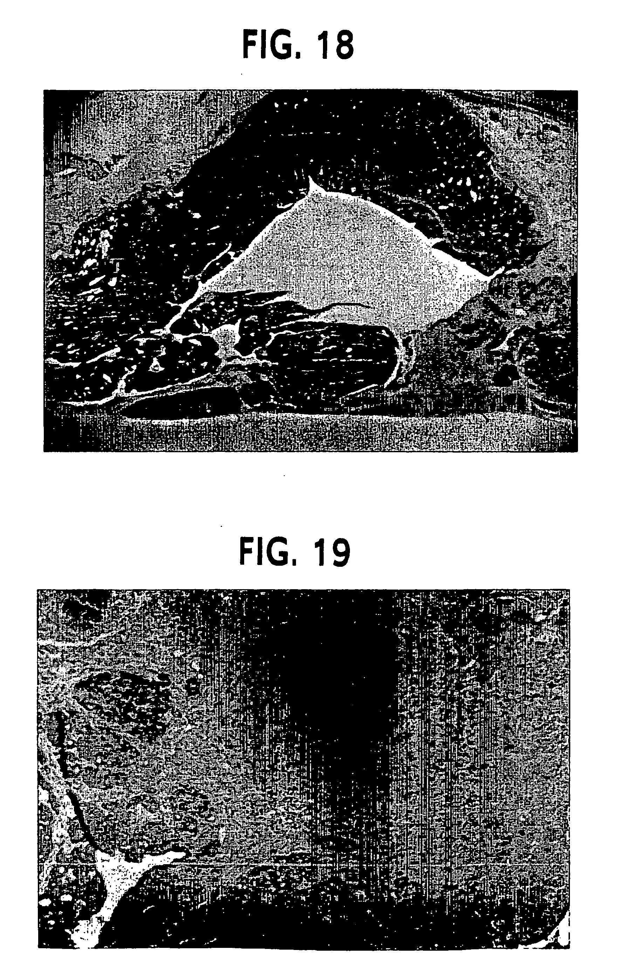

- Figure 18:: Shows tumor necrosis 2 weeks after AD7C-NTP injection. Optical microscopy, hematoxylin and eosin stain x40; and
- Figure 19:: Shows tumor necrosis 2 weeks after AD7C- NTP injection. Optical microscopy, hematoxylin and eosin stain x200.

### DETAILED DESCRIPTION OF THE PREFERRED EMBODIMENTS

### Definitions

Terms and phrases used herein are defined as set forth below unless otherwise specified.

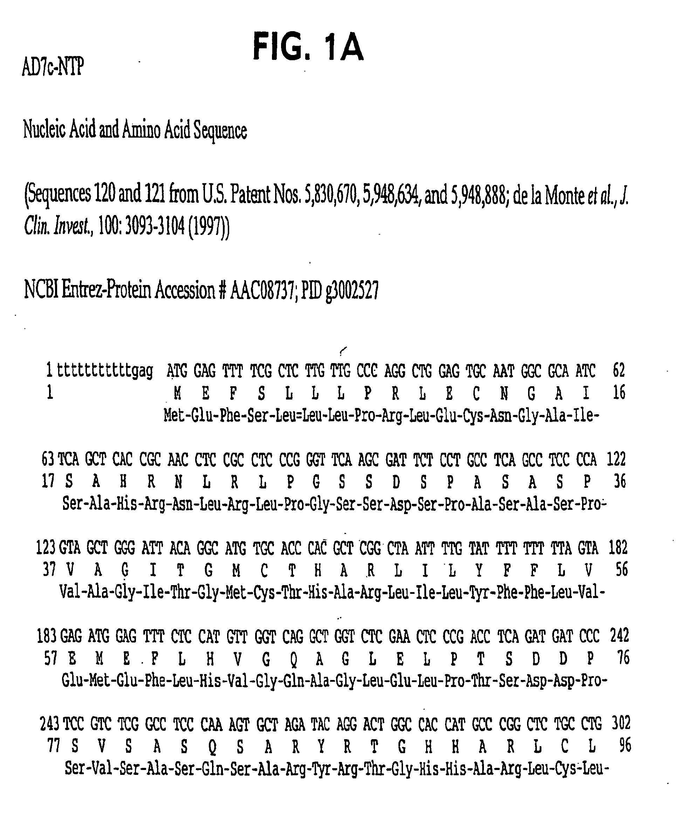

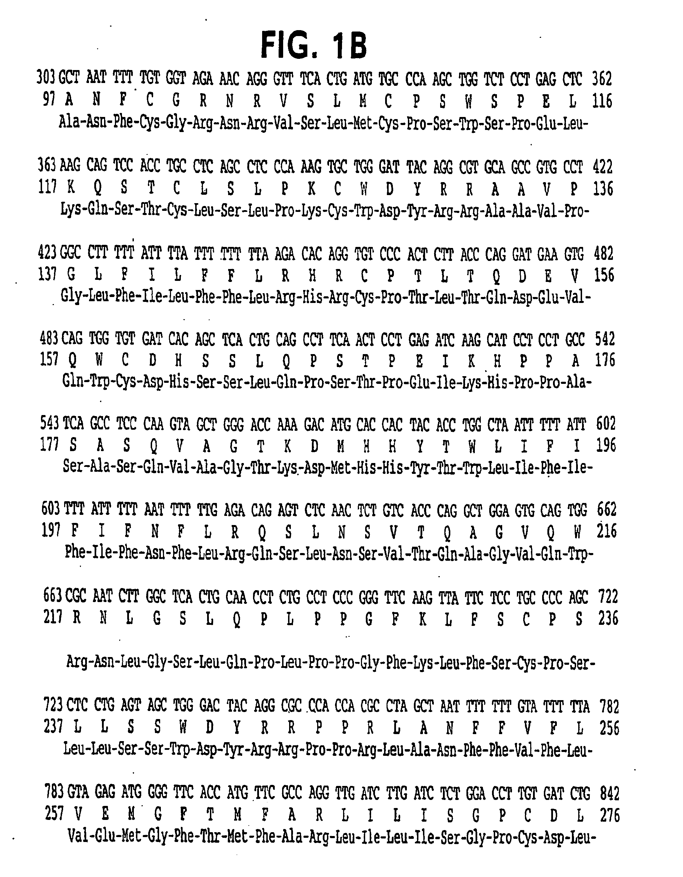

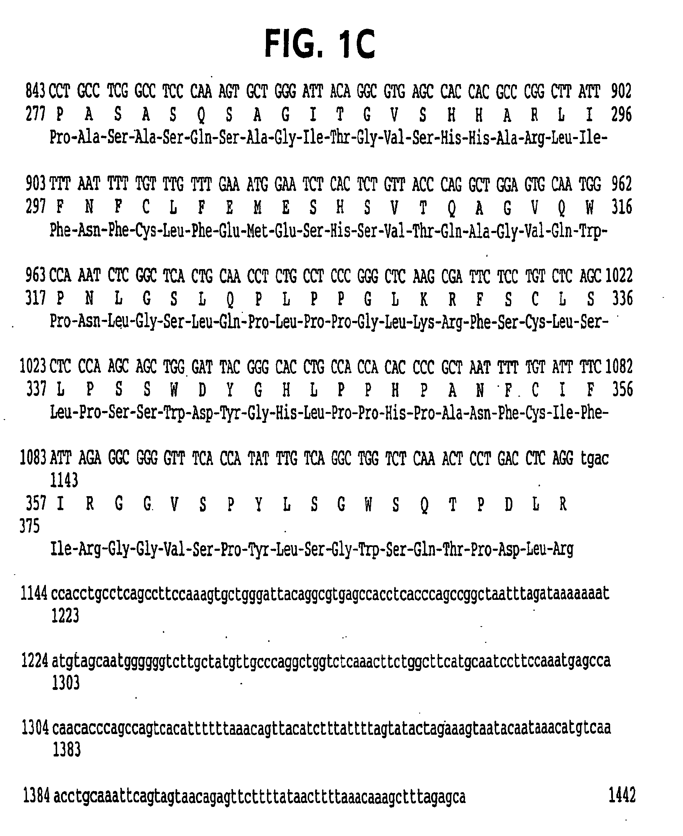

The term "AD7c-NTP" refers to the ~41kD protein and the gene and the nucleic acid sequences coding for it described in de la Monte et al., J. Clin. Invest., 100:3093-104 (1997), in Sequences 120 and 121 of U.S. Patent Nos. 5,948,634, 5,948,888, and 5,830,670 and in GenBank #AF010144, the nucleic acid and amino acid sequences for which are illustrated in Figure 1. The term "AD7c-NTP" also includes biologically active fragments, variants and derivatives, homologs, peptide mimetics, reverse-D peptides, and enantiomers of AD7c-NTP.

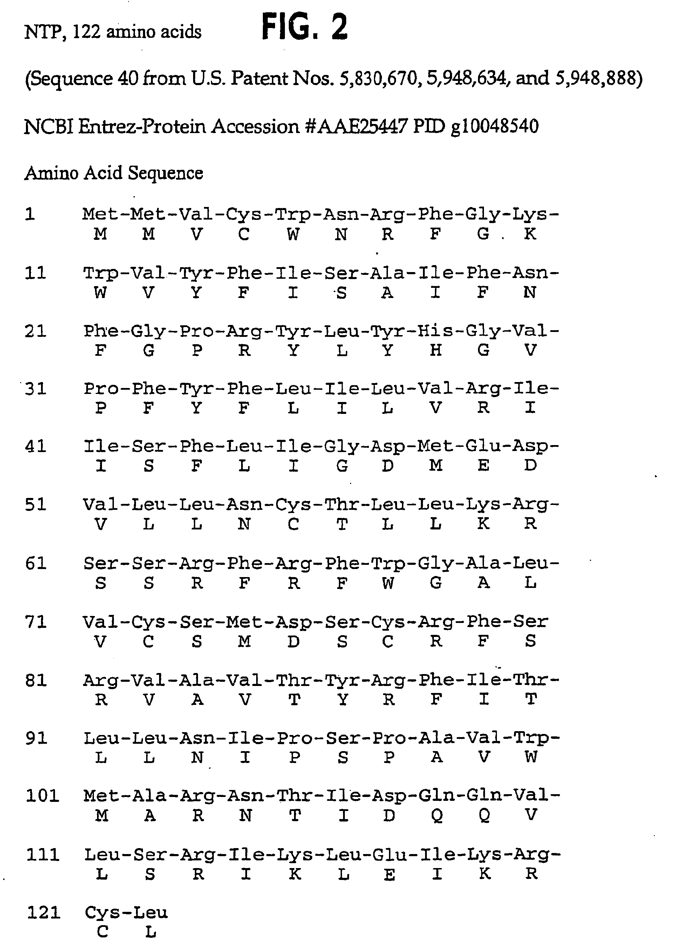

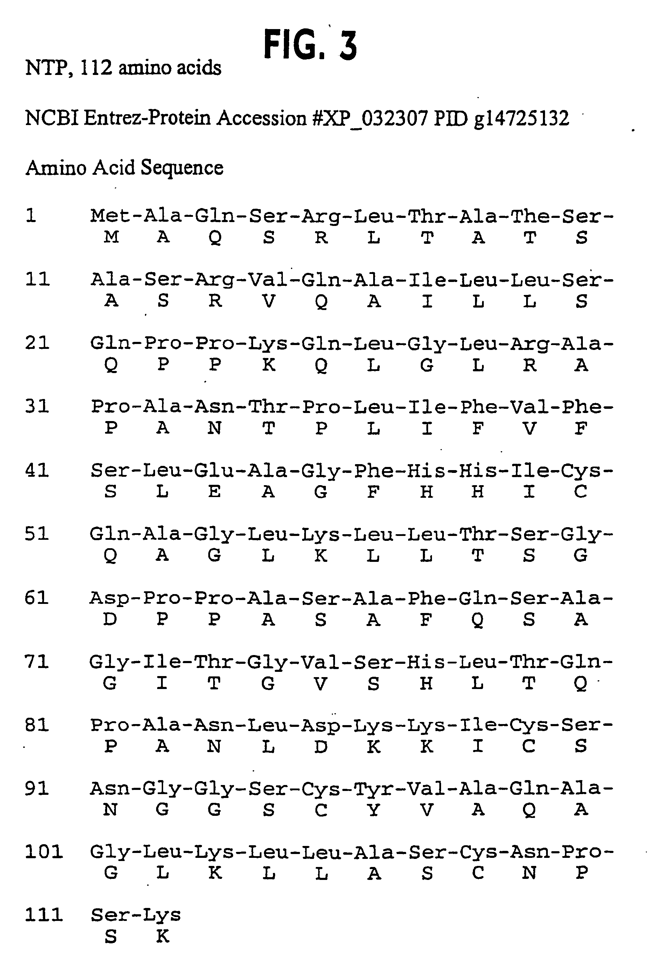

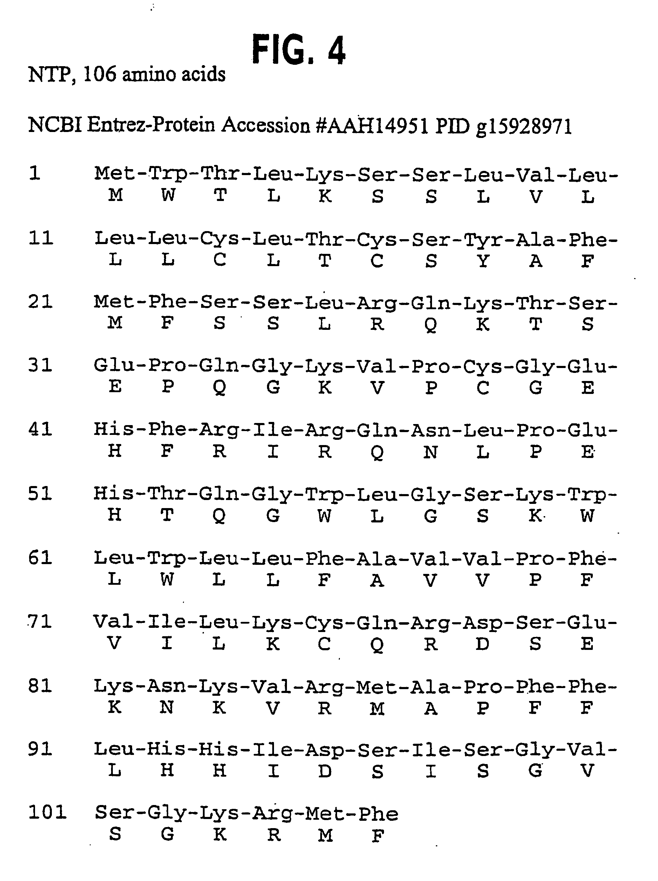

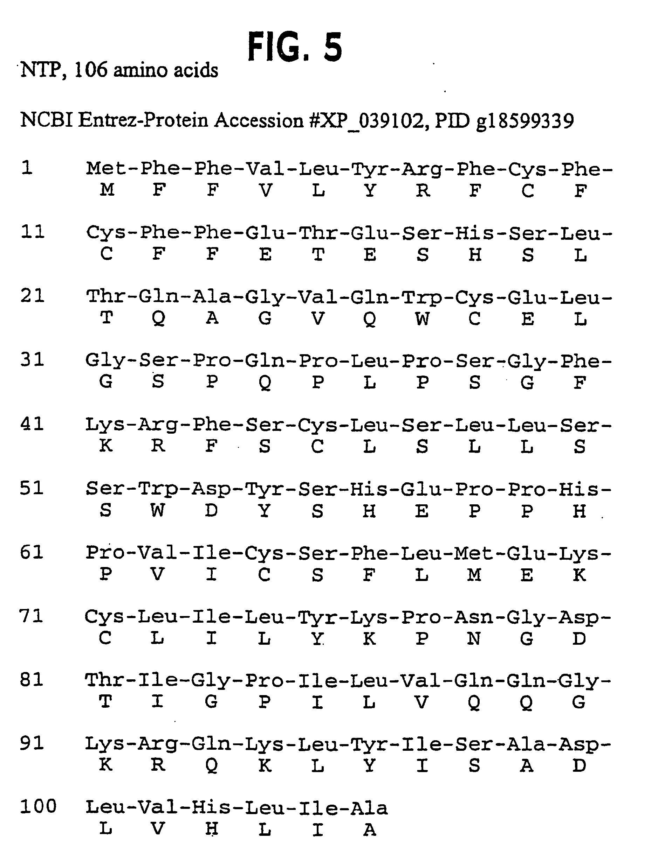

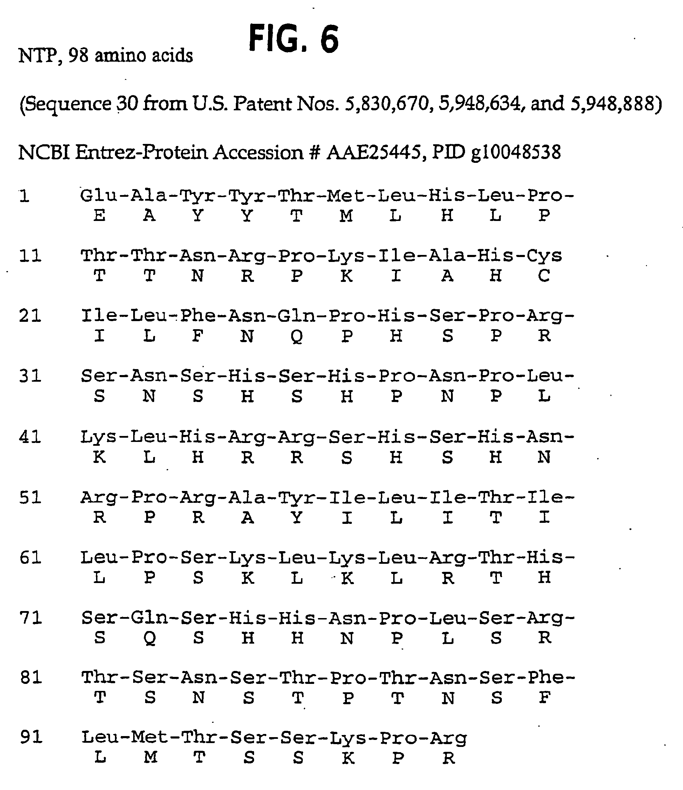

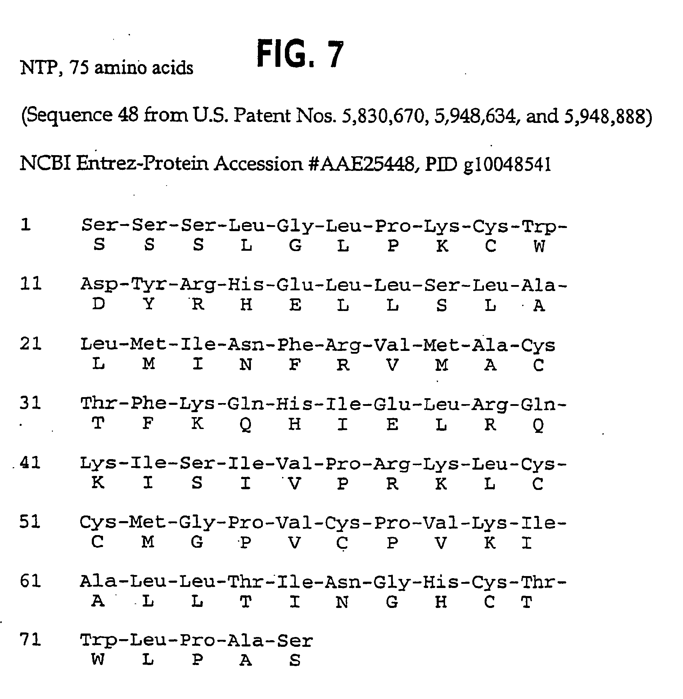

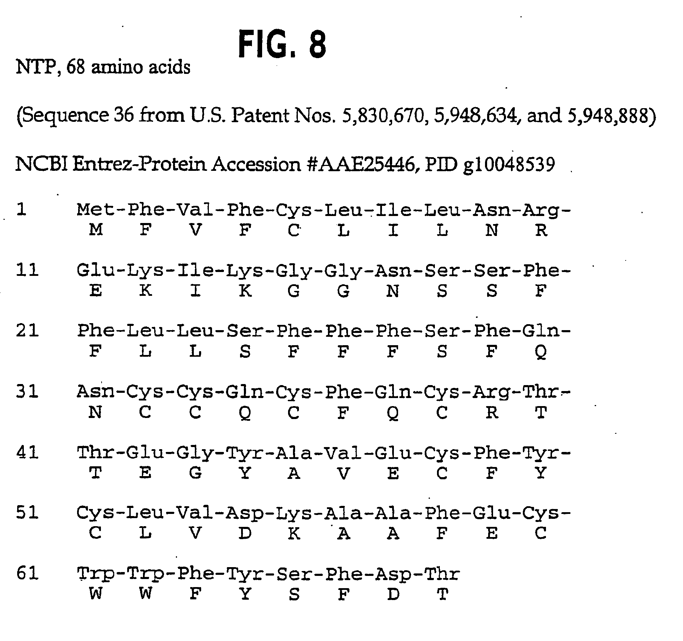

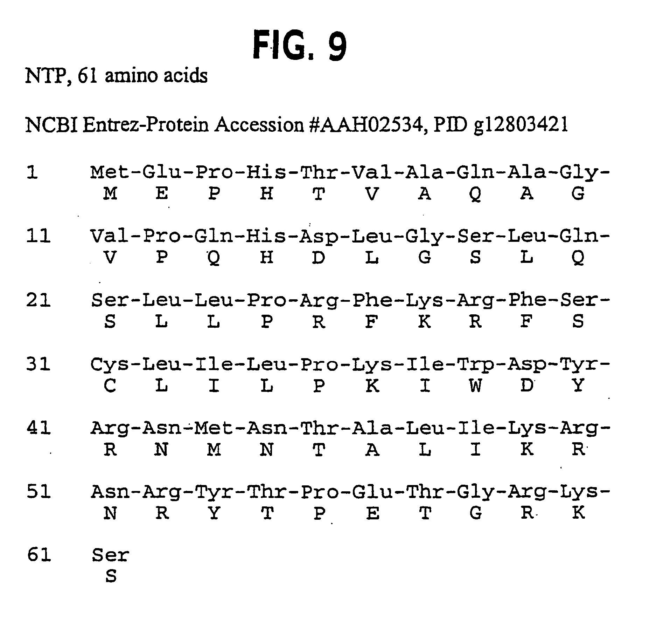

The term "NTP" or "neural thread protein" refers to neural thread proteins and related molecules (including pancreatic thread protein) and the nucleic acid sequences coding for those proteins, and includes (but is not limited to) the following proteins and the nucleic acid sequences encoding the amino acid sequences for these proteins:
(a) AD7c-NTP;
(b) the ∼42, ∼26, ∼21, ∼17, ∼14, and ∼8 kD species of neural thread protein as described in U.S. Patent Nos. 5,948,634, 5,948,888, 5,830,670, and 6,071,705 and in de la Monte et al., J. Neuropathol. Exp. Neurol., 55(10):1038-50 (1996), de la Monte et al., J. Neurol. Sci., 138(1-2):26-35 (1996); de la Monte et al., J. Neurol. Sci., 135(2):118-25 (1996), de la Monte et al., J. Clin. Invest., 100:3093-3104 (1997) and de la Monte et al., Alz.. Rep., 2:327-332 (1999);
(c) proteins specifically recognized by monoclonal antibody #2 on deposit with the American Type Culture Collection, Manassas, Va., under accession number HB-12546 or monoclonal antibody #5 on deposit with the American Type Culture Collection, Manassas, Va., under accession number HB-12545;
(d) proteins coded by the AD7c-NTP gene;
(e) the 122 amino acid neural thread protein described in Sequence 40 from U.S. Patent Nos. 5,830,670, 5,948,634, and 5,948,888 and listed in NCBI Entrez-Protein Accession #AAE25447, PID g10048540, the amino acid sequences for which is illustrated in Figure 2;
(f) the 112 amino acid neural thread protein listed in NCBI Entrez-Protein Accession #XP_032307, PID g14725132, the amino acid sequences for which is illustrated in Figure 3;
(g) a 106 amino acid neural thread protein-like protein listed in NCBI Entrez-Protein Accession #AAH14951 PID g15928971, the amino acid sequences for which is illustrated in Figure 4;
(h) a 106 amino acid neural thread protein-like protein listed in NCBI Entrez-Protein Accession #XP_039102, PID g18599339, the amino acid sequence for which is illustrated in Figure 5;
(i) the 98 amino acid neural thread protein described in Sequence 30 from U.S. Patent Nos. 5,830,670, 5,948,634, and 5,948,888 and listed in NCBI Entrez-Protein Accession # AAE25445, PID g10048538, the amino acid sequences for which is illustrated in Figure 6;
(j) the 75 amino acid neural thread protein described in Sequence 48 from U.S. Patent Nos. 5,830,670, 5,948,634, and 5,948,888 and listed in NCBI Entrez-Protein Accession #AAE25448, PID g10048541, the amino acid sequences for which is illustrated in Figure 7;
(k) the 68 amino acid neural thread protein described in Sequence 36 from U.S. Patent Nos. 5,830,670, 5,948,634, and 5,948,888 and listed in NCBI Entrez-Protein Accession #AAE25446, PID g10048539, the amino acid sequences for which is illustrated in Figure 8;
(l) the 61 amino acid neural thread protein-like protein listed in NCBI Entrez-Protein Accession #AAH02534, PID g12803421, the amino acid sequences for which is illustrated in Figure 9;
(m) pancreatic thread protein;
(n) the neural pancreatic thread protein (nPTP) described in U.S. Patent No. 6,071,705; and
(o) proteins specifically recognized by the antibodies produced by a hybridoma from the group consisting ofHB 9934, HB 9935, and HB 9936 deposited at the American Type Culture Collection.
The term "NTP" also includes biologically active fragments, variants and derivatives, homologs, peptide mimetics, reverse-D peptides, and enantiomers of NTP.

The term "biologically active" refers to a protein, polypeptide fragment, variant, derivative, homolog, reverse-D peptide, peptide mimetic, or enantiomer which has the ability of destroying and hence either facilitating the removal of or inhibiting the further growth of harmful or unwanted cells and tissue but having mainly local effects and minimal or absent systemic toxicity and includes (but not limited to) the ability to kill or decrease the viability of cells in the cell culture cytotoxicity assay of Example 1 herein, the ability to cause cell loss and/or tissue necrosis in the *in vivo* tissue assay of Example 2 herein, or the ability to cause cell loss, tissue necrosis or tumor shrinkage in the tumor assay of Example 3 herein.

The term "fragment" refers to a biologically active protein or polypeptide that consists of a continuous subsequence of the amino acid sequence of an NTP protein or variant, derivative, homolog, reverse-D peptide or enantiomer thereof and includes naturally occurring fragments such as splice variants and fragments resulting from naturally occurring *in vivo* protease activity. Such a fragment may be truncated at the amino terminus, the carboxy terminus, and/or internally (such as by natural splicing), and may be a variant or a derivative of an NTP protein. Such fragments may be prepared with or without an amino terminal methionine. The term "fragment" includes fragments, whether identical or different, from the same NTP protein or with a contiguous amino acid sequence in common or not, joined together, either directly or through a linker.

The term "variant" refers to a biologically active protein or polypeptide in which one or more amino acid substitutions, deletions, and/or insertions are present as compared to the amino acid sequence of an NTP protein and includes naturally occurring allelic variants or alternative splice variants of an NTP protein. The term "variant" includes the replacement of one or more amino acids in a peptide sequence with a similar or homologous amino acid(s) or a dissimilar amino acid(s). There are many scales on which amino acids can be ranked as similar or homologous. (Gunnar von Heijne, Sequence Analysis in Molecular Biology, p. 123-39 (Academic Press, New York, NY 1987.) Preferred variants include alanine substitutions at one or more of amino acid positions. Other preferred substitutions include conservative substitutions that have little or no effect on the overall net charge, polarity, or hydrophobicity of the protein. Conservative substitutions are set forth in Table I below.

**TABLE I**

| Conservative Amino Acid Substitutions | |
|---|---|
| Basic: | arginine |
| | lysine |
| | histidine |
| Acidic: | glutamic acid |
| | aspartic acid |
| Uncharged Polar: | glutamine |
| | asparagine |
| | serine |
| | threonine |
| | tyrosine |
| Non-Polar: | phenylalanine |
| | tryptophan |
| | cysteine |
| | glycine |
| | alanine |
| | valine |
| | proline |
| | methionine |
| | leucine |
| | isoleucine |

Table II sets out another scheme of amino acid substitution:

**TABLE II**

| Original Residue | Substitutions |
|---|---|
| Ala | gly; ser |
| Arg | lys |
| Asn | gln; his |
| Asp | glu |
| Cys | ser |
| Gln | asn |
| Glu | asp |
| Gly | ala; pro |
| His | asn; gln |
| Ile | leu; val |
| Leu | ile; val |
| Lys | arg; gln; glu |
| Met | leu; tyr; ile |
| Phe | met; leu; tyr |
| Ser | thr |
| Thr | ser |
| Trp | tyr |
| Tyr | trp; phe |
| Val | ile; leu |

Other variants can consist of less conservative amino acid substitutions, such as selecting residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions that in general are expected to have a more significant effect on function are those in which (a) glycine and/or proline is substituted by another amino acid or is deleted or inserted; (b) a hydrophilic residue, e.g., seryl or threonyl, is substituted for (or by) a hydrophobic residue, e.g., leucyl, isoleucyl, phenylalanyl, valyl, or alanyl; (c) a cysteine residue is substituted for (or by) any other residue; (d) a residue having an electropositive side chain, e.g., lysyl, arginyl, or histidyl, is substituted for (or by) a residue having an electronegative charge, e.g., glutamyl or aspartyl; or (e) a residue having a bulky side chain, e.g., phenylalanine, is substituted for (or by) one not having such a side chain, e.g., glycine. Other variants include those designed to either generate a novel glycosylation and/or phosphorylation site(s), or those designed to delete an existing glycosylation and/or phosphorylation site(s). Variants include at least one amino acid substitution at a glycosylation site, a proteolytic cleavage site and/or a cysteine residue. Variants also include NTP proteins, fragments, derivatives, homologs, reverse-D peptides and antimers with additional amino acid residues before or after the NTP amino acid sequence on linker peptides. For example, a cysteine residue may be added at both the amino and carboxy terminals of an NTP fragment in order to allow the cyclisation of the NTP fragment by the formation of a di-sulphide bond. The term "variant" also includes variants of fragments, derivatives, homologs, reverse-D peptides and enantiomers of NTP or AD7c-NTP.

The term "derivative" refers to a biologically active, chemically modified protein or polypeptide that have been chemically modified either by natural processes, such as processing and other post-translational modifications, but also by chemical modification techniques, as for example, by addition of one or more polyethylene glycol molecules, sugars, phosphates, and/or other such molecules, where the molecule or molecules are not naturally attached to wild-type NTP proteins. Derivatives include salts. Such chemical modifications are well described in basic texts and in more detailed monographs, as well as in a voluminous research literature, and they are well known to those of skill in the art. It will be appreciated that the same type of modification may be present in the same or varying degree at several sites in a given protein or polypeptide. Also, a given protein or polypeptide may contain many types of modifications. Modifications can occur anywhere in a protein or polypeptide, including the peptide backbone, the amino acid side-chains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, glycosylation, lipid attachment, sulfation, gamma-carboxylation of glutamic acid residues, hydroxylation and ADP-ribosylation, selenoylation, sulfation, transfer-RNA mediated addition of amino acids to proteins, such as arginylation, and ubiquitination. See, for instance, Proteins-Structure And Molecular Properties; 2nd Ed., T. E. Creighton, W. H. Freeman and Company, New York (1993) and Wold, F., "Posttranslational Protein Modifications: Perspectives and Prospects," pgs. 1-12 in Posttranslational Covalent Modification Of Proteins, B. C. Johnson, Ed., Academic Press, New York (1983); Seifter et al., Meth. Enzymol. 182:626-646 (1990) and Rattan et al., "Protein Synthesis: Posttranslational Modifications and Aging," Ann. N.Y. Acad. Sci. 663: 48-62 (1992). The term "derivatives" include chemical modifications resulting in the protein or polypeptide becoming branched or cyclic, with or without branching. Cyclic, branched and branched circular proteins or polypeptides may result from post-translational natural processes and may be made by entirely synthetic methods, as well. The term "derivative" also includes derivatives of fragments, variants, homologs, reverse-D peptides, peptide mimetics, and enantiomers.

The term "homolog" refers to a biologically active protein that is at least 75 percent identical in its amino acid sequence of an NTP protein or of AD7c-NTP, as the case may be, as determined by standard methods that are commonly used to compare the similarity in position of the amino acids of two polypeptides. The degree of similarity or identity between two proteins can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991; and Carillo H. and Lipman, D., SIAM, J. Applied Math., 48: 1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research, 12(1): 387 (1984)), BLASTP, BLASTN, and FASTA, Atschul, S. F. et al., J. Molec. Biol., 215: 403-410 (1990). The ***BLAST*** X program is publicly available from NCBI and other sources (***BLAST*** Manual, Altschul, S., et al., NCBI NLM NIH Bethesda, Md. 20894; Altschul, S., et al., J. Mol. Biol., 215: 403-410 (1990). By way of example, using a computer algorithm such as GAP (Genetic Computer Group, University of Wisconsin, Madison, Wis.), the two proteins or polypeptides for which the percent sequence identity is to be determined are aligned for optimal matching of their respective amino acids (the "matched span", as determined by the algorithm). A gap opening penalty (which is calculated as 3.times.the average diagonal; the "average diagonal" is the average of the diagonal of the comparison matrix being used; the "diagonal" is the score or number assigned to each perfect amino acid by the particular comparison matrix) and a gap extension penalty (which is usually 1/10 times the gap opening penalty), as well as a comparison matrix such as PAM 250 or BLOSUM 62 are used in conjunction with the algorithm. A standard comparison matrix (see Dayhoff et al. in: Atlas of Protein Sequence and Structure, vol. 5, supp.3 [1978] for the PAM250 comparison matrix; see Henikoff et al., Proc. Natl. Acad. Sci USA, 89:10915-10919 [1992] for the BLOSUM 62 comparison matrix) is also used by the algorithm. The percent identity is then calculated by the algorithm. Homologs will typically have one or more amino acid substitutions, deletions, and/or insertions as compared with NTP or AD7c-NTP.

The term "peptide mimetic" refers to biologically active compounds that mimic the biological activity of a peptide or a protein but are no longer peptidic in chemical nature, that is, they no longer contain any peptide bonds (that is, amide bonds between amino acids). Here the term peptide mimetic is used in a broader sense to include molecules that are no longer completely peptidic in nature, such as pseudo-peptides, semi-peptides and peptoids. Examples of peptide mimetics in this broader sense (where part of a peptide is replaced by a structure lacking peptide bonds) are described below. Whether completely or partially non-peptide, peptide mimetics according to this invention provide a spatial arrangement of reactive chemical moieties that closely resemble the three-dimensional arrangement of active groups in the NTP protein on which the peptide mimetic is based. As a result of this similar active-site geometry, the peptide, mimetic has effects on biological systems that are similar to the biological activity of the peptide. The peptide mimetics of this invention are preferably substantially similar in both three-dimensional shape and biological activity to the NTP peptides set forth above. Examples of methods of structurally modifying a peptide known in the art to create a peptide mimetic include the inversion of backbone chiral centers leading to D-amino acid residue structures that may, particularly at the N-terminus, lead to enhanced stability for proteolytical degradation without adversely affecting activity. An example is given in the paper "Tritriated D-ala.sup.1 -Peptide T Binding", Smith C. S. et al., Drug Development Res., 15, pp. 371-379 (1988). A second method is altering cyclic structure for stability, such as N to C interchain imides and lactames (Ede et al. in Smith and Rivier (Eds.) "Peptides: Chemistry and Biology", Escom, Leiden (1991), pp. 268-270). An example of this is given in conformationally restricted thymopentin-like compounds, such as those disclosed in U.S. Pat. No. 4,457,489 (1985), Goldstein, G. et al.*,* the disclosure of which is incorporated by reference herein in its entirety. A third method is to substitute peptide bonds in the NTP peptide by pseudopeptide bonds that confer resistance to proteolysis. A number of pseudopeptide bonds have been described that in general do not affect peptide structure and biological activity. One example of this approach is to substitute retro-inverso pseudopeptide bonds ("Biologically active retroinverso analogues of thymopentin", Sisto A. et al in Rivier, J. E. and Marshall, G. R (eds) "Peptides, Chemistry, Structure and Biology", Escom, Leiden (1990), pp. 722-773) and Dalpozzo, et al. (1993), Int. J. Peptide Protein Res., 41:561-566). According to this modification, the amino acid sequences of the peptides may be identical to the sequences of the NTP peptides described above, except that one or more of the peptide bonds are replaced by a retro-inverso pseudopeptide bond. Preferably the most N-terminal peptide bond is substituted, since such a substitution will confer resistance to proteolysis by exopeptidases acting on the N-terminus. Further modifications also can be made by replacing chemical groups of the amino acids with other chemical groups of similar structure. Another suitable pseudopeptide bond that is known to enhance stability to enzymatic cleavage with no or little loss of biological activity is the reduced isostere pseudopeptide bond is a (Couder, et al. (1993), Int. J. Peptide Protein Res., 41:181-184). Thus, the amino acid sequences of these peptides may be identical to the sequences of an NTP protein, except that one or more of the peptide bonds are replaced by an isostere pseudopeptide bond. Preferably the most N-terminal peptide bond is substituted, since such a substitution would confer resistance to proteolysis by exopeptidases acting on the N-terminus. The synthesis of peptides with one or more reduced isostere pseudopeptide bonds is known in the art (Couder, *et al.* (1993), cited above). Other examples include the introduction of ketomethylene or methylsulfide bonds to replace peptide bonds.

Peptoid derivatives of NTP peptides represent another class of peptide mimetics that retain the important structural determinants for biological activity, yet eliminate the peptide bonds, thereby conferring resistance to proteolysis (Simon, et al., 1992, Proc. Natl. Acad Sci. USA, 89:9367-9371). Peptoids are oligomers of N-substituted glycines. A number of N-alkyl groups have been described, each corresponding to the side chain of a natural amino acid (Simon, *et al.* (1992), cited above). Some or all of the amino acids of the NTP are replaced with the N-substituted glycine corresponding to the replaced amino acid.

The term "reverse-D peptide" refers to a biologically active protein or peptide consisting ofD-amino acids arranged in a reverse order as compared to the L-amino acid sequence of an NTP protein, fragment, variant or homolog. Thus, the carboxy terminal residue of an L-amino acid NTP protein, fragment, variant or homolog becomes the amino terminal for the D-amino acid peptide and so forth. For example, the AD7c-NTP fragment, HVGQAG, becomes G_{d}A_{d}Q_{d}G_{d}V_{d}H_{d}, where A_{d}, G_{d}, H_{d}, Q_{d}, and V_{d} are the D-amino acids corresponding to the L-amino acids, A, G, H, Q, and V respectively.

The term "enantiomer" refers to a biologically active protein or peptide where one or more the L-amino acid residues in the amino acid sequence of an NTP protein, fragment, variant or homolog is replaced with the corresponding D-amino acid residue(s).

Amino acids and amino acid residues described herein may be referred to according to the accepted one or three-letter code provided in the table below. Unless otherwise specified, these amino acids or residues are of the naturally occurring L stereoisomer form.

**Table III**

| Amino Acid | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

### Preparation of NTP Proteins and Fragments

NTP proteins (including AD7c-NTP, fragments, variants, derivatives, and homologs) encompassed by this invention can be prepared using methods known to those of skilled in the art, such as recombinant DNA technology, protein synthesis and isolation of naturally occurring NTP andAD7c-NTP and fragments and variants thereof.

An NTP protein can be prepared using well known recombinant DNA technology methods such as those set forth in Sambrook et al. (Molecular Cloning: A Laboratory Manual, Cold Spring Harbor Laboratory Press, Cold Spring Harbor, N.Y. [1989]) and/or Ausubel et al., eds., *Current Protocols in Molecular Biology,* Green Publishers Inc. and Wiley and Sons, N.Y. [1994].

A gene or cDNA encoding an NTP protein may be obtained for example by screening a genomic or cDNA library, or by PCR amplification. Probes or primers useful for screening the library can be generated based on sequence information for other known genes or gene fragments from the same or a related family of genes, such as, for example, conserved motifs found in other NTP proteins. In addition, where a gene encoding an NTP protein has been identified from one species, all or a portion of that gene may be used as a probe to identify homologous genes from other species. The probes or primers may be used to screen cDNA libraries from various tissue sources believed to express an NTP gene. Typically, conditions of high stringency will be employed for screening to minimize the number of false positives obtained from the screen.

Another means to prepare a gene encoding an NTP protein is to employ chemical synthesis using methods well known to the skilled artisan, such as those described by Engels et al.(Angew. Chem. Intl. Ed., 28:716-734 [1989]). These methods include, inter alia, the phosphotriester, phosphoramidite, and H-phosphonate methods for nucleic acid synthesis. A preferred method for such chemical synthesis is polymer-supported synthesis using standard phosphoramidite chemistry. Typically, the DNA encoding an NTP protein will be several hundred nucleotides in length. Nucleic acids larger than about 100 nucleotides can be synthesized as several fragments using these methods. The fragments can then be ligated together to form the full length NTP protein. Usually, the DNA fragment encoding the amino terminus of the protein will have an ATG, which encodes a methionine residue. This methionine may or may not be present on the mature form of the NTP protein, depending on whether the protein produced in the host cell is designed to be secreted from that cell.

The gene, cDNA, or fragment thereof encoding the NTP protein can be inserted into an appropriate expression or amplification vector using standard ligation techniques. The vector is typically selected to be functional in the particular host cell employed (i.e., the vector is compatible with the host cell machinery such that amplification of the gene and/or expression of the gene can occur). The gene, cDNA or fragment thereof encoding the NTP protein may be amplified/expressed in prokaryotic, yeast, insect (baculovirus systems) and/or eukaryotic host cells. Selection of the host cell will depend in part on whether the NTP protein is to be glycosylated and/or phosphorylated. If so, yeast, insect, or mammalian host cells are preferable.

Typically, the vectors used in any of the host cells will contain 5' flanking sequence (also referred to as a "promoter") and other regulatory elements as well such as an enhancer(s), an origin of replication element, a transcriptional termination element, a complete intron sequence containing a donor and acceptor splice site, a signal peptide sequence, a ribosome binding site element, a polyadenylation sequence, a polylinker region for inserting the nucleic acid encoding the polypeptide to be expressed, and a selectable marker element. Each of these elements is discussed below. Optionally, the vector may contain a "tag" sequence, i.e., an oligonucleotide molecule located at the 5' or 3' end of the NTP protein coding sequence; the oligonucleotide molecule encodes polyHis (such as hexaHis), or other "tag" such as FLAG, HA (hemaglutinin Influenza virus) or myc for which commercially available antibodies exist. This tag is typically fused to the polypeptide upon expression of the polypeptide, and can serve as means for affinity purification of the NTP protein from the host cell. Affinity purification can be accomplished, for example, by column chromatography using antibodies against the tag as an affinity matrix. Optionally, the tag can subsequently be removed from the purified NTP protein by various means such as using certain peptidases.

The human immunoglobulin hinge and Fc region could be fused at either the N-terminus or C-terminus of the NTP protein by one skilled in the art. The subsequent Fc-fusion protein could be purified by use of a Protein A affinity column. Fc is known to exhibit a long pharmacokinetic half-life in vivo and proteins fused to Fc have been found to exhibit a substantially greater half-life in vivo than the unfused counterpart. Also, fusion to the Fc region allows for dimerization/multimerization of the molecule that may be useful for the bioactivity of some molecules.

The 5' flanking sequence may be homologous (i.e., from the same species and/or strain as the host cell), heterologous (i.e., from a species other than the host cell species or strain), hybrid (i.e., a combination of 5' flanking sequences from more than one source), synthetic, or it may be the native NTP protein gene 5' flanking sequence. As such, the source of the 5' flanking sequence may be any unicellular prokaryotic or eukaryotic organism, any vertebrate or invertebrate organism, or any plant, provided that the 5' flanking sequence is functional in, and can be activated by, the host cell machinery.

The 5' flanking sequences useful in the vectors of this invention may be obtained by any of several methods well known in the art. Typically, 5' flanking sequences useful herein other than the NTP gene flanking sequence will have been previously identified by mapping and/or'by restriction endonuclease digestion and can thus be isolated from the proper tissue source using the appropriate restriction endonucleases. In some cases, the full nucleotide sequence of the 5' flanking sequence may be known. Here, the 5' flanking sequence may be synthesized using the methods described above for nucleic acid synthesis or cloning.

Where all or only a portion of the 5' flanking sequence is known, it may be obtained using PCR and/or by screening a genomic library with suitable oligonucleotide and/or 5' flanking sequence fragments from the same or another species.

Where the 5' flanking sequence is not known, a fragment of DNA containing a 5' flanking sequence may be isolated from a larger piece of DNA that may contain, for example, a coding sequence or even another gene or genes. Isolation may be accomplished by restriction endonuclease digestion using one or more carefully selected enzymes to isolate the proper DNA fragment. After digestion, the desired fragment may be isolated by agarose gel purification, Qiagen.RTM. column or other methods known to the skilled artisan. Selection of suitable enzymes to accomplish this purpose will be readily apparent to one of ordinary skill in the art.

The origin of replication element is typically a part of prokaryotic expression vectors purchased commercially, and aids in the amplification of the vector in a host cell. Amplification of the vector to a certain copy number can, in some cases, be important for optimal expression of the NTP protein. If the vector of choice does not contain an origin of replication site, one may be chemically synthesized based on a known sequence, and ligated into the vector. The transcription termination element is typically located 3' of the end of the NTP protein coding sequence and serves to terminate transcription of the NTP protein. Usually, the transcription termination element in prokaryotic cells is a G-C rich fragment followed by a poly T sequence. While the element is easily cloned from a library or even purchased commercially as part of a vector, it can also be readily synthesized using methods for nucleic acid synthesis such as those described above.

A selectable marker gene element encodes a protein necessary for the survival and growth of a host cell grown in a selective culture medium. Typical selection marker genes encode proteins that (a) confer resistance to antibiotics or other toxins, e.g., ampicillin, tetracycline, or kanamycin for prokaryotic host cells, (b) complement auxotrophic deficiencies of the cell; or (c) supply critical nutrients not available from complex media. Preferred selectable markers are the kanamycin resistance gene, the ampicillin resistance gene, and the tetracycline resistance gene.

The ribosome binding element, commonly called the Shine-Dalgarno sequence (prokaryotes) or the Kozak sequence (eukaryotes), is usually necessary for translation initiation of mRNA. The element is typically located 3' to the promoter and 5' to the coding sequence of the NTP protein to be synthesized. The Shine-Dalgarno sequence is varied but is typically a polypurine (i.e., having a high A-G content). Many Shine-Dalgarno sequences have been identified, each of which can be readily synthesized using methods set forth above and used in a prokaryotic vector.

In those cases where it is desirable for NTP protein to be secreted from the host cell, a signal sequence may be used to direct the NTP protein out of the host cell where it is synthesized, and the carboxy-terminal part of the protein may be deleted in order to prevent membrane anchoring. Typically, the signal sequence is positioned in the coding region of the NTP gene or cDNA, or directly at the 5' end of the NTP gene coding region. Many signal sequences have been identified, and any of them that are functional in the selected host cell may be used in conjunction with the NTP gene or cDNA. Therefore, the signal sequence may be homologous or heterologous to the NTP gene or cDNA, and may be homologous or heterologous to the NTP gene or cDNA. Additionally, the signal sequence may be chemically synthesized using methods set forth above. In most cases, secretion of the polypeptide from the host cell via the presence of a signal peptide will result in the removal of the amino terminal methionine from the polypeptide.

In many cases, transcription of the NTP gene or cDNA is increased by the presence of one or more introns in the vector; this is particularly true where the NTP protein is produced in eukaryotic host cells, especially mammalian host cells. The introns used may be naturally occurring within the NTP gene, especially where the gene used is a full length genomic sequence or a fragment thereof. Where the intron is not naturally occurring within the gene (as for most cDNAs), the intron(s) may be obtained from another source. The position of the intron with respect to the flanking sequence and the NTP gene is generally important, as the intron must be transcribed to be effective. As such, where the NTP gene inserted into the expression vector is a cDNA molecule , the preferred position for the intron is 3' to the transcription start site, and 5' to the polyA transcription termination sequence. Preferably for NTP cDNA, the intron or introns will be located on one side or the other (i.e., 5' or 3') of the cDNA such that it does not interrupt the this coding sequence. Any intron from any source, including any viral, prokaryotic and eukaryotic (plant or animal) organisms, may be used to practice this invention, provided that it is compatible with the host cell(s) into which it is inserted. Also included herein are synthetic introns. Optionally, more than one intron may be used in the vector.

Where one or more of the elements set forth above are not already present in the vector to be used, they may be individually obtained and ligated into the vector. Methods used for obtaining each of the elements are well known to the skilled artisan and are comparable to the methods set forth above (i.e., synthesis of the DNA, library screening, and the like).

The final vectors used to practice this invention are typically constructed from a starting vectors such as a commercially available vector. Such vectors may or may not contain some of the elements to be included in the completed vector. If none of the desired elements are present in the starting vector, each element may be individually ligated into the vector by cutting the vector with the appropriate restriction endonuclease(s) such that the ends of the element to be ligated in and the ends of the vector are compatible for ligation. In some cases, it may be necessary to "blunt" the ends to be ligated together in order to obtain a satisfactory ligation. Blunting is accomplished by first filling in "sticky ends" using Klenow DNA polymerase or T4 DNA polymerase in the presence of all four nucleotides. This procedure is well known in the art and is described for example in Sambrook et al., supra. Alternatively, two or more of the elements to be inserted into the vector may first be ligated together (if they are to be positioned adjacent to each other) and then ligated into the vector.

One other method for constructing the vector to conduct all ligations of the various elements simultaneously in one reaction mixture. Here, many nonsense or nonfunctional vectors will be generated due to improper ligation or insertion of the elements, however the functional vector may be identified and selected by restriction endonuclease digestion.

Preferred vectors for practicing this invention are those which are compatible with bacterial, insect, and mammalian host cells. Such vectors include, inter alia, pCRII, pCR3, and pcDNA3.1 (Invitrogen Company, San Diego, Calif.), pBSII (Stratagene Company, La Jolla, Calif.), pET15b (Novagen, Madison, Wis.), PGEX (Pharmacia Biotech, Piscataway, N.J.), pEGFP-N2 (Clontech, Palo Alto, Calif.), pETL (BlueBacII; Invitrogen), and pFastBacDual (Gibco/BRL, Grand Island, N.Y.).

After the vector has been constructed and a nucleic acid molecule encoding full length or truncated NTP protein has been inserted into the proper site of the vector, the completed vector may be inserted into a suitable host cell for amplification and/or polypeptide expression. Host cells may be prokaryotic host cells (such as E. coli) or eukaryotic host cells (such as a yeast cell, an insect cell, or a vertebrate cell). The host cell, when cultured under appropriate conditions, can synthesize NTP proteino which can subsequently be collected from the culture medium (if the host cell secretes it into the medium) or directly from the host cell producing it (if it is not secreted).

After collection, the NTP protein can be purified using methods such as molecular sieve chromatography, affinity chromatography, and the like. Selection of the host cell for NTP protein production will depend in part on whether the NTP protein is to be glycosylated or phosphorylated (in which case eukaryotic host cells are preferred), and the manner in which the host cell is able to "fold" the protein into its native tertiary structure (e.g., proper orientation of disulfide bridges, etc.) such that biologically active protein is prepared by the NTP protein that has biological activity, the NTP protein may be "folded" after synthesis using appropriate chemical conditions as discussed below. Suitable cells or cell lines may be mammalian cells, such as Chinese hamster ovary cells (CHO), human embryonic kidney (HEK) 293 or 293T cells, or 3T3 cells. The selection of suitable mammalian host cells and methods for transformation, culture, amplification, screening and product production and purification are known in the art. Other suitable mammalian cell lines, are the monkey COS-1 and COS-7 cell lines, and the CV-1 cell line. Further exemplary mammalian host cells include primate cell lines and rodent cell lines, including transformed cell lines. Normal diploid cells, cell strains derived from in vitro culture of primary tissue, as well as primary explants, are also suitable. Candidate cells may be genotypically deficient in the selection gene, or may contain a dominantly acting selection gene. Other suitable mammalian cell lines include but are not limited to, mouse neuroblastoma N2A cells, HeLa, mouse L-929 cells, 3T3 lines derived from Swiss, Balb-c or NIH mice, BHK or HaK hamster cell lines.

Similarly useful as host cells suitable for the present invention are bacterial cells. For example, the various strains of *E. coli* (e.g., HB101, DH5.alpha., DH10, and MC1061) are well-known as host cells in the field of biotechnology. Various strains of *B. subtilis,* Pseudomonas spp., other Bacillus spp., Streptomyces spp., and the like may also be employed in this method. Many strains of yeast cells known to those skilled in the art are also available as host cells for expression of the polypeptides of the present invention.

Additionally, where desired, insect cell systems may be utilized in the methods of the present invention. Such systems are described for example in Kitts et al. (Biotechniques, 14:810-817 [1993]), Lucklow (Curr. Opin. Biotechnol., 4:564-572 [1993]) and Lucklow et al. (J. Virol., 67:4566-4579 [1993]). Preferred insect cells are Sf-9 and Hi5 (Invitrogen, Carlsbad, Calif.).

Insertion (also referred to as "transformation" or "transfection") of the vector into the selected host cell may be accomplished using such methods as calcium chloride, electroporation, microinjection, lipofection, or the DEAE-dextran method. The method selected will in part be a function of the type of host cell to be used. These methods and other suitable methods are well known to the skilled artisan, and are set forth, for example, in Sambrook et al., *supra.*

The host cells containing the vector (i.e., transformed or transfected) may be cultured using standard media well known to the skilled artisan. The media will usually contain all nutrients necessary for the growth and survival of the cells. Suitable media for culturing E. coli cells are for example, Luria Broth (LB) and/or Terrific Broth (TB). Suitable media for culturing eukaryotic cells are RPMI 1640, MEM, DMEM, all of which may be supplemented with serum and/or growth factors as required by the particular cell line being cultured. A suitable medium for insect cultures is Grace's medium supplemented with yeastolate, lactalbumin hydrolysate, and/or fetal calf serum as necessary. Typically, an antibiotic or other compound useful for selective growth of the transformed cells only is added as a supplement to the media. The compound to be used will be dictated by the selectable marker element present on the plasmid with which the host cell was transformed. For example, where the selectable marker element is kanamycin resistance, the compound added to the culture medium will be kanamycin.

The amount of NTP protein produced in the host cell can be evaluated using standard methods known in the art. Such methods include, without limitation, Western blot analysis, SDS-polyacrylamide gel electrophoresis, non-denaturing gel electrophoresis, HPLC separation, immunoprecipitation, and/or activity assays such as DNA binding gel shift assays.

If the NTP protein has been designed to be secreted from the host cells, the majority of the NTP protein may be found in the cell culture medium. Proteins prepared in this way will typically not possess an amino terminal methionine, as it is removed during secretion from the cell. If however, the NTP protein is not secreted from the host cells, it will be present in the cytoplasm and/or the nucleus (for eukaryotic host cells) or in the cytosol (for gram negative bacteria host cells) and may have an amino terminal methionine.

For NTP protein situated in the host cell cytoplasm and/or nucleus, the host cells are typically first disrupted mechanically or with detergent to release the intra-cellular contents into a buffered solution. NTP protein can then be isolated from this solution.

Purification of NTP protein from solution can be accomplished using a variety of techniques. If the protein has been synthesized such that it contains a tag such as hexaHistidine (NTP protein/hexaHis) or other small peptide such as FLAG (Sigma-Aldritch, St. Louis, MI) or calmodulin-binding peptide (Stratagene, La Jolla, CA) at either its carboxyl or amino terminus, it may essentially be purified in a one-step process by passing the solution through an affinity column where the column matrix has a high affinity for the tag or for the protein directly (i.e., a monoclonal antibody specifically recognizing the NTP protein). For example, polyhistidine binds with great affinity and specificity to nickel, zinc and cobalt thus immobilized metal ion affinity chromatography which employs a nickel-based affinity resin (as used in Qiagen's QIAexpress system or Invitrogen's Xpress System) or a cobalt-based affinity resin (as used in BD Biosciences-CLONTECH's Talon system) can be used for purification of NTP protein/polyHis. (See for example, Ausubel et al., eds., Current Protocols in Molecular Biology, Section 10.11.8, John Wiley & Sons, New York [1993]).

Where the NTP protein is prepared without a tag attached, and no antibodies are available, other well known procedures for purification can be used. Such procedures include, without limitation, ion exchange chromatography, hydroxyapatite chromatography, hydrophobic interaction chromatography, molecular sieve chromatography, HPLC, native gel electrophoresis in combination with gel elution, and preparative isoelectric focusing ("Isoprime" machine/technique, Hoefer Scientific). In some cases, two or more of these techniques may be combined to achieve increased purity.

If it is anticipated that the NTP protein will be found primarily intracellularly, the intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication followed by centrifugation. If the NTP protein has formed inclusion bodies in the cytosol, the inclusion bodies can often bind to the inner and/or outer cellular membranes and thus will be found primarily in the pellet material after centrifugation. The pellet material can then be treated at pH extremes or with chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The NTP protein in its now soluble form can then be analyzed using gel electrophoresis, immunoprecipitation or the like. If it is desired to isolate the NTP protein, isolation may be accomplished using standard methods such as those set forth below and in Marston et al. (Meth. Enz., 182:264-275 [1990]). In some cases, the NTP protein may not be biologically active upon isolation. Various methods for "refolding" or converting the polypeptide to its tertiary structure and generating disulfide linkages, can be used to restore biological activity. Such methods include exposing the solubilized polypeptide to a pH usually above 7 and in the presence of a particular concentration of a chaotrope. The selection of chaotrope is very similar to the choices used for inclusion body solubilization but usually at a lower concentration and is not necessarily the same chaotrope as used for the solubilization. In most cases the refolding/oxidation solution will also contain a reducing agent or the reducing agent plus its, oxidized form in a specific ratio to generate a particular redox potential allowing for disulfide shuffling to occur in the formation of the protein's cysteine bridge(s). Some of the commonly used redox couples include cysteine/cystamine, glutathione (GSH)/dithiobis GSH, cupric chloride, dithiothreitol(DTT)/dithiane DTT, 2-mercaptoethanol(bME)/dithio-b(ME). In many instances a cosolvent is necessary to increase the efficiency of the refolding and the more common reagents used for this purpose include glycerol, polyethylene glycol of various molecular weights, and arginine.

If NTP protein inclusion bodies are not formed to a significant degree in the host cell, the NTP protein will be found primarily in the supernatant after centrifugation of the cell homogenate, and the NTP protein can be isolated from the supernatant using methods such as those set forth below.

In those situations where it is preferable to partially or completely isolate the NTP protein, purification can be accomplished using standard methods well known to the skilled artisan. Such methods include, without limitation, separation by electrophoresis followed by electroelution, various types of chromatography (immunoaffinity, molecular sieve, and/or ion exchange), and/or high pressure liquid chromatography. In some cases, it may be preferable to use more than one of these methods for complete purification.

In addition to preparing and purifying NTP protein using recombinant DNA techniques, the NTP proteins and their derivatives may be prepared by chemical synthesis methods (such as solid phase peptide synthesis) using techniques known in the art such as those set forth by Merrifield et al., (J. Am. Chem. Soc., 85:2149 [1963]), Houghten et al. (Proc Natl Acad. Sci. USA, 82:5132 [1985]), and Stewart and Young (Solid Phase Peptide Synthesis, Pierce Chemical Co., Rockford, I11. [1984]). Such polypeptides may be synthesized with or without a methionine on the amino terminus. Chemically synthesized NTP proteins may be oxidized using methods set forth in these references to form disulfide bridges. The NTP proteins are expected to have biological activity comparable to NTP proteins produced recombinantly or purified from natural sources, and thus may be used interchangeably with recombinant or natural NTP protein.

Chemically modified NTP protein compositions in which the NTP protein is linked to a polymer are included within the scope of the present invention. The polymer selected is typically water soluble so that the protein to which it is attached does not precipitate in an aqueous environment, such as a physiological environment. The polymer selected is usually modified to have a single reactive group, such as an active ester for acylation or an aldehyde for alkylation, so that the degree of polymerization may be controlled as provided for in the present methods. The polymer may be of any molecular weight, and may be branched or unbranched. Included within the scope of NTP protein polymers is a mixture of polymers.

In some cases, it may be desirable to prepare nucleic acid and/or amino acid variants of the naturally occurring NTP proteins. Nucleic acid variants may be produced using site directed mutagenesis, PCR amplification, or other appropriate methods, where the primer(s) have the desired point mutations (see Sambrook et al., supra, and Ausubel et al., supra, for descriptions of mutagenesis techniques). Chemical synthesis using methods described by Engels et al., supra, may also be used to prepare such variants. Other methods known to the skilled artisan may be used as well.

Preferred nucleic acid variants are those containing nucleotide substitutions accounting for codon preference in the host cell that is to be used to produce the NTP protein. Such "codon optimization" can be determined via computer algorithers which incorporate codon frequency tables such as "Ecohigh. Cod" for codon preference of highly expressed bacterial genes as provided by the University of Wisconsin Package Version 9.0, Genetics Computer Group, Madison, Wis. Other useful codon frequency tables include "Celegans_high.cod", "Celegans _low.cod", Drosophila_high.cod", "Human_high.cod", "Maize_high.cod", and "Yeast_high.cod". Other preferred variants are those encoding conservative amino acid changes as described above (e.g., wherein the charge or polarity of the naturally occurring amino acid side chain is not altered substantially by substitution with a different amino acid) as compared to wild type, and/or those designed to either generate a novel glycosylation and/or phosphorylation site(s), or those designed to delete an existing glycosylation and/or phosphorylation site(s).

NTP fragments, homologs, variants, derivatives and salts thereof can be made using conventional peptide synthesis techniques known to one of ordinary skill in the art. These techniques include chemical coupling methods (cf. Wunsch, E: "Methoden der organischen Chemie", Volume 15, Band 1+2, Synthese von Peptiden, thime Verlag, Stuttgart (1974), and Barrany, G.; Marrifield, R. B.: "The Peptides", eds. E. Gross, J. Meienhofer, Volume 2, Chapter 1, pp. 1-284, Academic Press (1980)), enzymatic coupling methods (cf. Widmer, F. Johansen, J. T., Carlsberg Res. Commun., Vol. 44, pp. 37-46 (1979), and Kullmann, W.: "Enzymatic Peptide Synthesis", CRC Press Inc. Boca Raton, Fla. (1987), and Widmer, F., Johansen, J. T. in "Synthetic Peptides in Biology and Medicines:, eds. Alitalo, K., Partanen, P., Vatieri, A., pp.79-86, Elsevier, Amsterdam (1985)), or a combination of chemical and enzymatic methods if this is advantageous for the process design and economy. Those skilled in the art are capable of varying the peptide sequence of the NTP protein to make a homolog having the same or similar biological activity (bioactivity) as the original or native NTP protein..

There are clear advantages for using a mimetic of a given NTP protein rather than the protein itself, because proteins commonly exhibit two undesirable properties: (1) poor bioavailability; and (2) short duration of action. Peptide mimetics offer a route around these two major obstacles, since the molecules concerned are small enough to be both more widely available and have a long duration of action. Furthermore, peptide mimetics are much cheaper to produce than proteins and peptides. Finally, there are problems associated with stability, storage, and immunoreactivity for proteins and peptides that are not experienced with peptide mimetics.

Thus the NTP peptides described above have utility in the development of such small chemical compounds with similar biological activities and therefore with similar therapeutic utilities. Peptide mimetics of NTP peptides can be developed using combinatorial chemistry techniques and other techniques known in the art (see e.g. Proceedings of the 20th European Peptide Symposium, ed. G. Jung, E. Bayer, pp. 289-336, and references therein).

Examples of methods of structurally modifying a peptide known in the art to create a peptide mimetic include the inversion of backbone chiral centers leading to D-amino acid residue structures that may, particularly at the N-terminus, lead to enhanced stability for proteolytical degradation without adversely affecting activity. An example is given in the paper "Tritriated D-ala.sup.1 -Peptide T Binding", Smith C. S. et al., Drug Development Res. 15, pp. 371-379 (1988).

A second method is altering cyclic structure for stability, such as N to C interchain imides and lactames (Ede et al. in Smith and Rivier (Eds.) "Peptides: Chemistry and Biology", Escom, Leiden (1991), pp. 268-270). An example of this is given in conformationally restricted thymopentin-like compounds, such as those disclosed in U.S. Pat. No. 4,457,489 (1985), Goldstein, G. et al.

A third method is to substitute peptide bonds in the NTP peptide by pseudopeptide bonds that confer resistance to proteolysis. A number of pseudopeptide bonds have been described that in general do not affect peptide structure and biological activity. One example of this approach is to substitute retro-inverso pseudopeptide bonds ("Biologically active retroinverso analogues of thymopentin", Sisto A. et al in Rivier, J. E. and Marshall, G. R. (eds) "Peptides, Chemistry, Structure and Biology", Escom, Leiden (1990), pp. 722-773) and Dalpozzo, et al. (1993), Int. J. Peptide Protein Res., 41:561-566). According to this modification, the amino acid sequences of the peptides may be identical to the sequences of the NTP peptides described above, except that one or more of the peptide bonds are replaced by a retro-inverso pseudopeptide bond. Preferably the most N-terminal peptide bond is substituted, since such a substitution will confer resistance to proteolysis by exopeptidases acting on the N-terminus.

The synthesis of peptides with one or more reduced retro-inverso pseudopeptide bonds is known in the art (Sisto (1990) and Dalpozzo, *et al.* (1993), cited above). Thus, peptide bonds can be replaced by non-peptide bonds that allow the peptide mimetic to adopt a similar structure, and therefore biological activity, to the original peptide. Further modifications also can be made by replacing chemical groups of the amino acids with other chemical groups of similar structure. Another suitable pseudopeptide bond that is known to enhance stability to enzymatic cleavage with no or little loss of biological activity is the reduced isostere pseudopeptide bond is a (Couder, et al. (1993), Int. J. Peptide Protein Res., 41:181-184). Thus, the amino acid sequences of these peptides may be identical to the sequences of an NTP peptide, except that one or more of the peptide bonds are replaced by an isostere pseudopeptide bond. Preferably the most N-terminal peptide bond is substituted, since such a substitution would confer resistance to proteolysis by exopeptidases acting on the N-terminus. The synthesis of peptides with one or more reduced isostere pseudopeptide bonds is known in the art (Couder, *et al.* (1993), cited above). Other examples include the introduction of ketomethylene or methylsulfide bonds to replace peptide bonds.

Peptoid derivatives ofNTP peptides represent another class of peptide mimetics that retain the important structural determinants for biological activity, yet eliminate the peptide bonds, thereby conferring resistance to proteolysis (Simon, et al., 1992, Proc. Natl. Acad. Sci. USA, 89:9367-9371). Peptoids are oligomers ofN-substituted glycines. A number of N-alkyl groups have been described, each corresponding to the side chain of a natural amino acid (Simon, *et al.* (1992), cited above). Some or all of the amino acids of the NTP are replaced with the N-substituted glycine corresponding to the replaced amino acid.

The development of peptide mimetics can be aided by determining the tertiary structure of the original NTP peptide by NMR spectroscopy, crystallography and/or computer-aided molecular modeling. These techniques aid in the development of novel compositions of higher potency and/or greater bioavailability and/or greater stability than the original peptide (Dean (1994), BioEssays, 16: 683-687; Cohen and Shatzmiller (1993), J. Mol. Graph., 11: 166-173; Wiley and Rich (1993), Med. Res. Rev., 13: 327-384; Moore (1994), Trends Pharmacol. Sci., 15: 124-129; Hruby (1993), Biopolymers, 33: 1073-1082; Bugg et al. (1993), Sci. Am., 269: 92-98).

Once a potential peptide mimetic compound is identified, it may be synthesized and assayed using the methods outlined in the examples below to assess its activity. The peptide mimetic compounds obtained by the above methods, having the biological activity of the NTP peptides and similar three-dimensional structure, are encompassed by this invention. It will be readily apparent to one skilled in the art that a peptide mimetic can be generated from any of the NTP peptides bearing one or more of the modifications described above. It will furthermore be apparent that the peptide mimetics of this invention can be further used for the development of even more potent non-peptidic compounds, in addition to their utility as therapeutic compounds.

### Conditions To Be Treated

The present invention is directed to use of therapeutically effective amount of a neural thread protein for the manufacture of a composition for treating tumors.

The tumors to be treated can be benign and malignant tumors, for example, tumors of long, breast, stomach, pancreas, prostate, bladder, bone, ovary, skin, kidney, sinus, colon, intestine, stomach, recturn, esophagus, blood, brain and its coverings, spinal cord and its coverings, muscle, connective tissue, adrenal, parathyroid, thyroid, uterus, testis, pituitary, reproductive organs, liver, gall bladder, eye, ear, nose, throat, tonsils, mouth, lymph nodes and lymphoid system, and other organs.

As used herein, the term "malignant tumor" is intended to encompass all forms of human carcinomas, sarcomas and melanomas which occur in the poorly differentiated, moderately differentiated, and well-differentiated forms.

This invention satisfies a need in the art for compositions that can remove benign tumors with less risk and fewer of the undesirable side effects of surgery. A composition for removing benign tumors in surgically hazardous areas such as in deep locations in the body (*e.g*., brain, heart, hmgs, and others) is particularly needed.

The composition for treating tumors can be used in conjunction with conventional methods of treating such conditions, such as surgical excision, chemotherapy, and radiation. NTP can be administered before, during, or after such conventional treatments.

### NTP Compositions

The use of NTP proteins for the manufacture of a composition are within the scope of the present invention. Such compositions may comprise a therapeutically effective amount of the NTP protein in admixture with a pharmaceutically acceptable carrier. The carrier material may be water for injection, preferably supplemented with other materials common in solutions for administration to mammals. Typically, an NTP protein for therapeutic use will be administered in the form of a composition comprising purified NTP protein in conjunction with one or more physiologically acceptable carriers, excipients, or diluents. Neutral buffered saline or saline mixed with serum albumin are exemplary appropriate carriers. Preferably, the product is formulated as a lyophilizate using appropriate excipients (e.g., sucrose). Other standard carriers, diluents, and excipients may be included as desired. Other exemplary compositions comprise Tris buffer of about pH 7.0-8.5, or acetate buffer of about pH 4.0-5.5, which may further include sorbitol or a suitable substitute therefor.

The use of NTP proteins conjugated or linked or bound to an antibody, antibody fragment, antibody-like molecule, or a molecule with a high affinity to a specific tumor marker, such as a cellular receptor, signal peptide or over-expressed enzyme, for targeting to the tumor is also encompassed by the scope of the invention. The antibody, antibody fragment, antibody-like molecule, or molecule with a high affinity to a specific tumor marker is used to target the NTP protein conjugate to a specific cellular or tissue target. For example, a tumor with a distinctive surface antigen or expressed antigen can be targeted by the antibody, antibody fragment, or antibody-like binding molecule and the tumor cells can be killed by the NTP protein. Such an approach using antibody targeting has the anticipated advantages of decreasing dosage, increasing the likelihood of binding to and uptake by the target cells, and increased usefulness for targeting and treating metastatic tumors and microscopic sized tumors.

This invention also encompasses the use of NTP proteins conjugated or linked or bound to a protein or other molecule to form a composition that, upon cleavage at or near the site(s) of the tumor or other unwanted cells by a tumor- or site-specific enzyme or protease or by an antibody conjugate that targets tumor, releases the NTP protein at or near the site(s) of the tumor or other unwanted cells

This invention also encompasses the use of NTP proteins conjugated or linked or bound to a protein or other molecule to form a composition that releases the NTP protein or some biologically active fragment of the NTP protein upon exposure of the tissue to be treated to light (as in laser therapies or other photo-dynamic or photo-activated therapy), other forms of electro-magnetic radiation such as infra-red radiation, ultraviolet radiation, x-ray or gamma ray radiation, localized heat, alpha or beta radiation, ultrasonic emissions, or other sources of localized energy.

The NTP proteins may be employed alone, together, or in combination with other pharmaceutical compositions, such as cytokines, growth factors, antibiotics, apoptotisinducing agents, anti-inflammatories, and/or chemotherapeutic agents as is appropriate for the indication being treated.

This invention also encompasses the use NTP for the manufacture of therapeutic compositions of NTP employing dendrimers, fullerenes, and other synthetic molecules, polymers and macromolecules where the NTP protein and/or its corresponding DNA molecule is conjugated with, attached to or enclosed in the molecule, polymer or macromolecule, either by itself or in conjunction with other species of molecule such as a tumor-specific marker. For example, U.S. Patent No. 5,714,166, *Bioactive and*/*or Targeted Dendimer Conjugates,* provides a method of preparing and using, *inter alia,* dendritic polymer conjugates composed of at least one dendrimer with a target director(s) and at least one bioactive agent conjugated to it.

Use of a therapeutically effective amount of an NTP gene for the manufacture of a composition for treating tumors is also encompassed by the scope of the invention. Overexpression of NTP within the tumor can be used to induce the cells in the tumor to die and thus reduce the tumor cell population. The gene or gene equivalent transfer of NTP to treat the tumor is anticipated to have the advantage of requiring less dosage, and of being passed on to the cellular progeny of the targeted cellular elements, thus necessitating less frequent therapy, and less total therapy.

The use of cloned recombinant NTP-antibody conjugates; cloned recombinant NTP-antibody fragment conjugates; and cloned recombinant NTP-antibody-like protein conjugates is also encompassed by the scope of the invention. The advantages of a cloned NTP combined with targeting conjugate (such as an antibody, antibody fragment, antibody-like molecule, or a molecule with a high affinity to a cancer-specific receptor or other tumor marker) are that such a molecule combines the targeting advantages described above in addition to advantages for manufacturing and standardized production of the cloned conjugated molecule.

### Dosage Forms

Solid dosage forms for oral administration include but are not limited to, capsules, tablets, pills, powders, and granules. In such solid dosage forms, the active compound is admixed with at least one of the following: (a) one or more inert excipients (or carrier), such as sodium citrate or dicalcium phosphate; (b) fillers or extenders, such as starches, lactose, sucrose, glucose, mannitol, and silicic acid; (c) binders, such as carboxymethylcellulose, alginates, gelatin, polyvinylpyrrolidone, sucrose and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as acetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active compounds, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

Actual dosage levels of active ingredients in the compositions of the invention may be varied to obtain an amount of NTP that is effective to obtain a desired therapeutic response for a particular composition and method of administration. The selected dosage level therefore depends upon the desired therapeutic effect, the route of administration, the desired duration of treatment, and other factors.

With mammals, including humans, the effective amounts can be administered on the basis of body surface area. The interrelationship of dosages for animals of various sizes, species and humans (based on mg/M² of body surface) is described by E. J. Freireich et al., Cancer Chemother. Rep., 50(4):219 (1966). Body surface area may be approximately determined from the height and weight of an individual (*see e.g.,* Scientific Tables, Geigy Pharmaceuticals, Ardsley, N.Y. pp. 537-538 (1970)).

The total daily dose of the NTP administered to a host may be in single or divided doses. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, potency of the administered drug, rates of absorption and excretion, combination with other drugs and the severity of the particular disease being treated.

### Methods of Administration

The NTP composition can be administered intramuscularly, orally, intravenously, intraperitoneally, intracerebrally (intraparenchymally), intracerebroventricularly, intratumorally, intralesionally, intradermally, intrathecally, intranasally, intraocularly, intraarterially, topically, transdermally, via an aerosol, infusion, bolus injection, implantation device, sustained release system etc.

Another of administering the NTP is by a transdermal or transcutaneous route. One example of such an embodiment is the use of a patch. In particular, a patch can be prepared with a fine suspension of NTP in, for example, dimethylsulfoxide (DMSO), or a mixture of DMSO with cottonseed oil and brought into contact with the skin of the tumor carrying mammals away from the tumor location site inside a skin pouch. Other mediums or mixtures thereof with other solvents and solid supports would work equally as well. The patch can contain the NTP compound in the form of a solution or a suspension. The patch can then be applied to the skin of the patient, for example, by means of inserting it into a skin pouch of the patient formed by folding and holding the skin together by means of stitches, clips or other holding devices. This pouch should be employed in such a manner so that continuous contact with the skin is assured without the interference of the mammal. Besides using a skin pouch, any device can be used which ensures the firm placement of the patch in contact with the skin. For instance, an adhesive bandage could be used to hold the patch in place on the skin.

NTP may be administered in a sustained release formulation or preparation. Suitable examples of sustained-release preparations include semipermeable polymer matrices in the form of shaped articles, e.g. films, or microcapsules. Sustained release matrices include polyesters, hydrogels, polylactides (U.S. 3,773,919, EP 58,481), copolymers of L-glutamic acid and gamma ethyl-L-glutamate (Sidman et al, Biopolymers, 22: 547-556 [1983]), poly (2-hydroxyethyl-methacrylate) (Langer et al.,J. Biomed. Mater. Res., 15: 167-277 [1981] and Langer, Chem. Tech., 12: 98-105 [1982]), ethylene vinyl acetate (Langer et al., supra) or poly-D(-)-3-hydroxybutyric acid (EP 133,988). Sustained-release compositions also may include liposomes, which can be prepared by any of several methods known in the art (e.g., Eppstein et al., Proc. Natl. Acad. Sci. USA, 82: 3688-3692 [1985]; EP 36,676; EP 88,046; and EP 143,949).

Another method of administering the NTP is by direct or indirect infusion ofNTP into the tumor. One example of such an embodiment is the direct injection of NTP into the tumor. The treatment may consist of a single injection. multiple injections on one occasion or a series of injections over a period of hours, days or months with the regression or destruction of the tumor being monitored by means of biopsy, imaging or other methods of monitoring tissue growth. The injection into the tumor may be by a device inserted into an orifice such as the nose, mouth, ear, vagina, rectum or urethra or through an incision in order to reach the tumor *in vivo* and may performed in conjunction with an imaging or optical system such as ultrasound or fibre optic scope in order to identify the appropriate site for the injection(s). Another example of such an embodiment is the use of a device that can provide a constant infusion of NTP to the tissue over time.

Another-method of administering the NTP is in conjunction with a surgical or similar procedure employed to physically excise, ablate or otherwise kill or destroy tumor wherein the NTP of the invention is administered to the immediate area(s) surrounding the area(s) where the tumor was removed in order to destroy or impede the growth of any tumor cells not removed or destroyed by the procedure

Another of administering the NTP is by implantation of a device within the tumor. One example of such an embodiment is the implantation of a wafer containing NTP in the tumor. The wafer releases a therapeutic dose of NTP into the tissue over time. Alternatively or additionally, the composition may be administered locally via implantation into the affected area of a membrane, sponge, or other appropriate material on to which the NTP of the invention has been absorbed. Where an implantation device is used, the device may be implanted into any suitable tissue or organ, and delivery of the NTP of the invention may be directly through the device via bolus, or via continuous administration, or via catheter using continuous infusion.

An alternative method of administration is to introduce one or more copies of the NTP gene into the cell being targeted and, if necessary, inducing the copy(ies) of the gene to begin producing NTP intracellularly. One manner in which gene therapy can be applied is to use the NTP gene (either genomic DNA, cDNA, and/or synthetic DNA encoding the NTP of this invention or a fragment, variant, or derivative thereof) which may be operably linked to a constitutive or inducible promoter to form a "gene therapy DNA construct". The promoter may be homologous or heterologous to the endogenous NTP gene, provided that it is active in the cell or tissue type into which the construct will be inserted. Other components of the gene therapy DNA construct may optionally include, as required, DNA molecules designed for site-specific integration (e.g., endogenous flanking sequences useful for homologous recombination), tissue-specific promoter, enhancer(s) or silencer(s), DNA molecules capable of providing a selective advantage over the parent cell, DNA molecules useful as labels to identify transformed cells, negative selection systems, cell specific binding agents (as, for example, for cell targeting) cell-specific internalization factors, and transcription factors to enhance expression by a vector as well as factors to enable vector manufacture.

Means of gene delivery to a cell or tissue *in vivo* or *ex* vivo include (but are not limited to) direct injection of bare DNA, ballistic methods, liposome-mediated transfer, receptor-mediated transfer (ligand-DNA complex), electroporation, and calcium phosphate precipitation. See U.S. Pat. Nos. 4,970,154, WO 96/40958, U.S. Pat. No. 5,679,559, U.S. Pat. No. 5,676,954, and U.S. Pat. No. 5,593,875. They also include use of a viral vector such as a retrovirus, adenovirus, adeno-associated virus, pox virus, lentivirus, papilloma virus or herpes simplex virus, use of a DNA-protein conjugate and use of a liposome. The use of gene therapy vectors is described, for example, in U.S. Pat. Nos. 5,672,344, U.S. Pat. No. 5,399,346, U.S. Pat. No.5,631,236, and U.S. Pat. No. 5,635,399.

The NTP gene may be delivered through implanting into patients certain cells that have been genetically engineered *ex vivo,* using methods such as those described herein, to express and secrete the NTP of this invention or fragments, variants, or derivatives thereof. Such cells may be animal or human cells, and may be derived from the patient's own tissue or from another source, either human or non-human. Optionally, the cells may be immortalized or be stem cells. However, in order to decrease the chance of an immunological response, it is preferred that the cells be encapsulated to avoid infiltration of surrounding tissues. The encapsulation materials are typically biocompatible, semi-permeable polymeric enclosures or membranes that allow release of the protein product(s) but prevent destruction of the cells by the patient's immune system or by other detrimental factors from the surrounding tissues. Methods used for membrane encapsulation of cells are familiar to the skilled artisan, and preparation of encapsulated cells and their implantation in patients may be accomplished without undue experimentation. See, e.g., U.S. Pat. Nos. 4,892,538; 5,011,472; and 5,106,627. A system for encapsulating living cells is described in PCT WO 91/10425. Techniques for formulating a variety of other sustained or controlled delivery means, such as liposome carriers, bio-erodible particles or beads, are also known to those in the art, and are described, for example, in U.S. Pat. No. 5,653,975. The cells, with or without encapsulation, may be implanted into suitable body tissues or organs of the patient.

The following examples are given to illustrate the present invention. It should be understood, however, that the invention is not to be limited to the specific conditions or details described in these examples.

### Example 1

The purpose of this example was to test the cytotoxic effect ofNTP on glioma and neuroblastoma cells.

Neuroblastoma is a cancer that primarily affects children. It begins in nerve tissue in the neck, chest, abdomen, or pelvis. It usually originates in the abdomen in the tissues of the adrenal gland. By the time it is diagnosed, the cancer often has spread, most commonly to the lymph nodes, liver, lungs, bones, and/or bone marrow.

Gliomas are brain tumors. A glioma tumor is particularly damaging because it tends to quickly sprout and spread within the brain. Each year, approximately 20,000 Americans find out that they have a glioma. More than half die within 18 months.

### Cell culture:

Cryopreserved glioma and neuroblastoma cells were acquired from the American Type Culture Collection (ATCC), Virginia. Cells were thawed and diluted in suspension media and centrifuged at 700 rpm for 7 minutes at 4°C. Cell pellets were then resuspended in CACO-2 media and cultured in standard 75 or 175 cm² flasks at 37°C, 5% CO₂ until approximately confluent. The cells were then trypsinized and resuspended in CACO-2 media to achieve a final cell density of 1.5 X 10⁵ cells/ml. 50 µl aliquots per sample were then added per well to 96 well plates.

### Dosing:

For Experiment I, 50 µl of either PBS (phosphate buffered solution) (negative control), 100 µM tamoxifen in phosphate buffered saline PBS (positive control), or test article were added to each sample culture.

For Experiment II, 1 mM tamoxifen in DMSO (dimethylsulfoxide) was diluted to 100 µM concentration in CACO-2 media, and 100 µl per well was added to positive control wells. A vehicle control consisting of 1% DMSO in CACO-2 media was also added to this experiment. Other negative controls consisted of human and bovine serum albumin and other nontoxic polypeptides.

### MTT assays:

An MTT assay is a sensitive assay for the measurement of cell proliferation based upon the reduction of the tetrazolium salt 3,[4,5-dimethylthiazol-2-yl]-2,5-diphenyltetrazolium bromide (MTT).

Following incubations with control or test substances, media was aspirated and 100 µl MTT was added to each well. Plates were then incubated for 3 hours at 37°C, 5% CO₂. MTT was then replaced with acidified isopropanol (0.4 N HCI) and the plates were stored overnight at 4°C, covered. Plates were then agitated gently for 1 minute on a rotary shaker and the difference between emission absorbance of 570 nm and background absorbance of 650 nm was measured spectrophotometrically on an automated plate reader.

### Results:

### Experiment I:

Neuroblastoma and glioma cells were incubated for 24 hours following treatment with test materials or control solutions. MTT was added to all cultures at this time. Results are expressed as means absorbance differences and represented as per cent of negative control and are shown in Table 1 (cytotoxicity in glioma cells) and Table 2 (cytotoxicity in neuroblastoma cells).

**Table 1: Cytotoxicity in glioma cells: 24 hrs.**

| Test Article Identification | Concentration | ABS 570-650 | | | Percent Of Viable Cells |
|---|---|---|---|---|---|
| | | Sample | Mean | SD | |
| NC (PBS) | | 0.064 | 0.061 | 0.005 | 100 |
| NC (PBS) | | 0.057 | | | |
| VC (DMSO) | | 0.051 | 0.054 | 0.004 | 100 |
| VC (DMSO) | | 0.056 | | | |
| PC (Tamoxifen) | 100 µM | 0.017 | 0.015 | 0.004 | 28 |
| PC (Tamoxifen) | 100 µM | 0.012 | | | |
| AD7C-NTP | 100-1000 ng/ml | 0.022 | 0.025 | 0.004 | 41 |
| AD7C-NTP | 100-1000 ng/ml | 0.028 | | | |

| | | | | | |
|---|---|---|---|---|---|
| Abbreviations: ABS, Absorbance; NC, Negative Control PC, Positive Control; SD, Standard Deviation; VC, Vehicle Control | | | | | |

**Table 2: Neuroblastoma cells: 24 hour incubation**

| Test Article Identification | Concentration | | | | Percent Of Viable Cells |
|---|---|---|---|---|---|
| | | Sample | Mean | SD | |
| | | 0.089 | 0.096 | 0.009 | 100 |
| NC (PBS) | | 0.102 | | | |
| VC (DMSO) | | 0.073 | 0.070 | 0.004 | 100 |
| VC (DMSO) | | 0.067 | | | |
| PC (Tamoxifen) | 100µM | 0.010 | 0.010 | 0.001 | 14 |
| PC (Tamoxifen) | 100µM | 0.009 | | | |
| AD7C-NTP | 100-1000 ng/ml | 0.012 | 0.013 | 0.001 | 14 |
| AD7C-NTP | 100-1000 ng/ml | 0.014 | | | |

| | | | | | |
|---|---|---|---|---|---|
| Abbreviations: ABS, Absorbance; NC, Negative Control PC, Positive Control; SD, Standard Deviation; VC, Vehicle Control | | | | | |

### Experiment 2:

Glioma cells and neuroblastma cells were used for this experiment. MTT was added to the plates following a 24 hour incubation with control or test solutions. Other plates were replenished with fresh media, control, or test solution at 24 hours and then read 3 days later. Results are expressed as mean absorbance differences and as per cent of negative control and are shown in Table 3 (glioma cells) and Table 4 (neuroblastoma cells).

**Table 3: Cytotoxicity in glioma cells: 4 days**

| Test Article Identification | Concentration | ABS 570-650 | | | Percent Of Viable Cells |
|---|---|---|---|---|---|
| | | Sample | Mean | SD | |
| NC (PBS) | | 0.107 | 0.093 | 0.016 | 100 |
| NC (PBS) | | 0.790 | | | |
| VC (DMSO) | | 0.076 | 0.079 | 0.004 | 100 |
| VC (DMSO) | | 0.081 | | | |
| PC (Tamoxifen) | 100µM | 0.018 | 0.015 | 0.003 | 19 |
| PC (Tamoxifen) | 100µM | 0.012 | | | |
| AD7C-NTP | 100-1000 ng/ml | 0.012 | 0.012 | 0.001 | 11 |
| AD7C-NTP | 100-1000 ng/ml | 0.011 | | | |

| | | | | | |
|---|---|---|---|---|---|
| Abbreviations: ABS, Absorbance; NC, Negative Control PC, Positive Control; SD, Standard Deviation; VC, Vehicle Control | | | | | |

**Table 4: Cytotoxicity effects of test articles on Neuroblastoma cells: 4 day incubation**

| Test Article Identification | Concentration | | | | Percent Of Viable Cells |
|---|---|---|---|---|---|
| | | Sample | Mean | SD | |
| NC (PBS) | | 0.088 | 0.022 | 0.022 | 100 |
| NC (PBS) | | 0.082 | | | |
| VC (DMSO) | | 0.076 | 0.076 | 0.001 | 100 |
| VC (DMSO) | | 0.077 | | | |
| PC (Tamoxifen) | 100µM | 0.016 | 0.019 | 0.003 | 25 |
| AD7C-NTP | 100-1000 ng/ml | 0.007 | 0.010 | 0.004 | 11 |
| AD7C-NTP | 100-1000 ng/ml | 0.012 | | | |

| | | | | | |
|---|---|---|---|---|---|
| Abbreviations: ABS, Absorbance; NC, Negative Control PC, Positive Control; SD, Standard Deviation; VC, Vehicle Control | | | | | |

### Conclusion:

Significant cytotoxic effects on glioma and neuroblastoma cells are apparent at 24 hours and at 96 hours with AD7C-NTP.

### Example 2

The purpose of this example was to determine the effect ofNTP on tissue at sites of injection.

Eight normal rats were injected in the skin and subcutaneously, each in 3 different foci, with purified recombinant AD7C-NTP in saline at concentrations of 0.1-10 µg/mL delivered from plastic syringes through stainless steel 26 gauge needles.

The animals were observed for 24 hours and painlessly sacrificed at 24 hours. The 24 individual foci of infiltration were excised, fixed in 10% formalin, embedded in paraffin, and stained and examined by standard histopathological methods.

Similar groups of control rats were injected with (1) bovine serum albumin 0.1% in saline, (2) normal human serum, (3) physiological saline, (4) *E. coli* proteins from vectors without AD7C-NTP, purified using procedures similar to the AD7C-NTP purification; (5) non-NTP recombinant protein expressed in *E. coli* and purified and then examined and sacrificed as above, with the excised foci of injection treated as above.

### Results

Injection of AD7C-NTP in all examples produced abundant acute necrosis of tissue at the injection sites (Figures 1-5). The necrosis is evident in muscle tissue, subcutaneous connective tissue, and dermis at the sites where the AD7C-NTP was injected. At 24 hours, cells appear pale, shrunken, and necrotic, and there is infiltration with inflammatory cells. The necrosis correlates with the areas of injection and does not appear to spread far beyond the site of injection.

Apart from the mild areas of inflammation, controls showed no evidence of necrosis or cell loss. In contrast to the AD7C-NTP injections where entire fields of muscle fiber layers were necrotic, the controls showed minimal or absent muscle changes. Control injections had mild to minimal acute inflammation at the injection sites and focal microhemorrhages from the needles.

### Example 3

The purpose of this example was to determine the effect of NTP on different human an non-human origin tumors.

AD7C-NTP infiltration was tested on a variety of different human and non-human origin tumors, including mammary carcinoma, skin carcinoma and papilloma, colon carcinoma, glioma of brain, and others in rodent models. The tumors were directly infiltrated with AD7C-NTP as described in Example 2 and the experimental animals were observed and painlessly sacrificed after intervals of 24 hours to 2 weeks. After sacrifice, histopathological examination of the tumor was performed as above.

Control tumors were tested with (1) no treatment, (2) infiltration with normal human serum, (3) infiltration with bovine serum albumin, and (4) infiltration with saline.

### Results

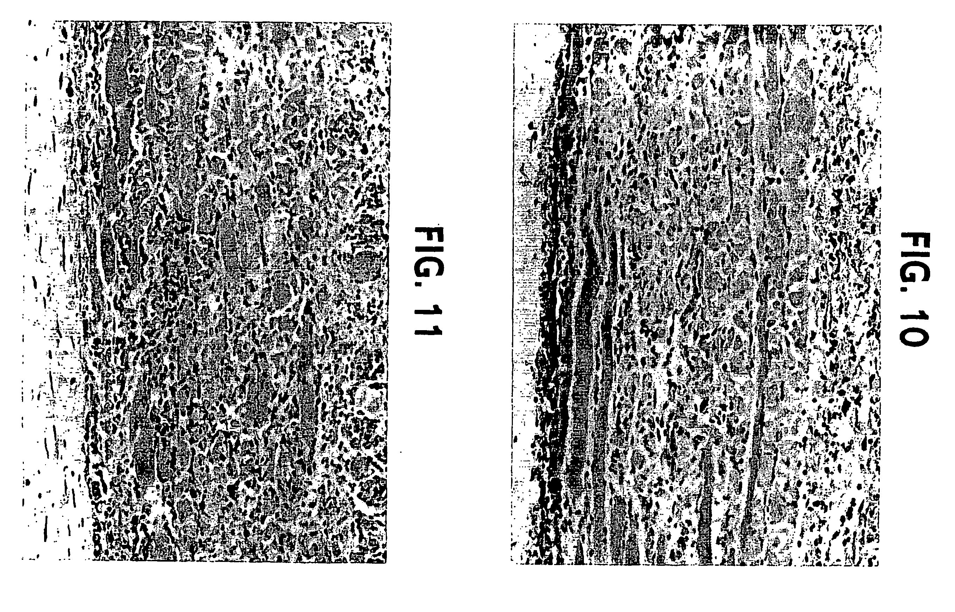

In all cases tested, there was significant necrosis of tumor cells after AD7C-NTP infiltration (Figures 6-10). In examples where there were two injections 24 hours apart, there was more tumor destruction than after a single injection. In some examples the tumor regressed almost completely. In other examples after a single injection, tumors grew larger 1-2 weeks later if there was no further injection of AD7C-NTP. In some tumors treated with one injection of AD7C-NTP and examined histologically two weeks later, there were large empty cavities where the tumor had previously been, surrounded by rims of tumor, indicating that the infiltrated tumor after necrosis had cavitated and disappeared. In other tumors treated with one injection and examined histologically two weeks later there was extensive fibrous within the tumor not seen in controls, attributed to tumor destruction and subsequent fibrosis. Control cases showed only focal microhemorrhages and inflammation and occasional distortion, hut insignificant necrosis and no evidence of the massive necrosis illustrated in the AD7C-NTP infusions.

## Claims

1. Use of a therapeutically effective amount of a neural thread protein (NTP) for the manufacture of a composition for treating tumors.

2. The use according to claim 1, wherein said NTP is to be administered by a method selected from the group consisting of orally, subcutaneously, intradermally, Intravenously, intramuscularly, intrathecally, intranasally, intratumorally, topically, and transdermally.

3. The use of any one of claims 1 or 2, wherein said treatment is to be administered to the patient before, during, or after treatment of the patient selected from the group of treatments consisting of surgical excision, transplantation, grafting, chemotherapy, immunotherapy, vaccination, thermal or electrical ablation, cryotherapy, laser therapy, phototherapy, gene therapy, and radiation.

4. The use of any one of claims 1 to 3, where the tumor is selected from the group consisting of a benign or malignant tumor of a tissue selected from the group consisting of lung, breast, stomach, pancreas, prostate, bladder, bone, ovary, skin, kidney, sinus, colon, intestine, stomach, rectum, esophagus, heart, spleen, salivary gland, blood, brain and its coverings, spinal cord and its coverings, muscle, connective tissue, adrenal, parathyroid, thyroid, uterus, testis, pituitary, reproductive organs, liver, gall bladder, eye, ear, nose, throat, mouth, and lymph nodes and lymphoid system.

5. The use of claim 4, wherein the tissue is selected from the group consisting of skin, eye, ear, nose, throat, mouth, muscle, connective tissue, hair, and breast.

6. The use of any one of claims 1 to 5, wherein the NTP is conjugated, linked, or bound to a molecule selected from the group consisting of an antibody, antibody fragment, and an antibody-like binding molecule, wherein said molecule has a higher affinity for binding to a tumor than binding to other cells.

7. The use of any one of claims 1 to 6, wherein the NTP is part of a single new cloned recombinant molecule consisting of NTP and a molecule selected from the group consisting of an antibody, antibody fragment, and antibody-like binding molecule, wherein said molecule has a higher affinity for binding to a tumor than binding to other cells.

8. The use of any of claims 1 to 7, wherein said NTP is chosen from the group consisting of AD7c-NTP (SEQ ID NO. 1), the proteins identified by SEQ ID NOs. 2 to 9, neural pancreatic thread protein and pancreatic thread protein.

9. The use according to claim 8, wherein said NTP is to be administered by a method selected from the group consisting of orally, subcutaneously, intradermally, intravenously, intramuscularly, intrathecally, intraperitoneally, intracerebrally (intraparenchymally), intracerebroventricularly, intraocularly, intraarterially, intranasally, intratumorally, intralesionally, topically, and transdermally.

10. The use of claim 8, wherein said treatment is to be administered to the patient before, during, or after treatment of the patient selected from the group of treatments consisting of surgical excision, transplantation, grafting, chemotherapy, immunotherapy, vaccination, thermal or electrical ablation, cryotherapy, laser therapy, phototherapy, gene therapy, and radiation.

11. The use of claim 8, where the tumor is a benign or malignant tumor of a tissue selected from the group consisting of lung, breast, stomach, pancreas, prostate, bladder, bone, ovary, skin, kidney, sinus, colon, intestine, stomach, rectum, esophagus, heart, spleen, salivary gland, blood, brain and its coverings, spinal cord and its coverings, muscle, connective tissue, adrenal, parathyroid, thyroid, uterus, testis, pituitary, reproductive organs, liver, gall bladder, eye, ear, nose, throat, tonsils, mouth, and lymph nodes and lymphoid system.

12. The use of claim 11, wherein the tissue is selected from the group consisting of skin, eye, ear, nose, throat, mouth, muscle, connective tissue, adipose tissue, hair, and breast.

13. The use of claim 8, wherein the said NTP is conjugated, linked, or bound to a molecule selected from the group consisting of an antibody, antibody fragment, and an antibody-like binding molecule, wherein said molecule, has a higher affinity for binding to a tumor than binding to other cells.

14. The use of claim 8, wherein the said NTP is part of a single new cloned recombinant molecule consisting of NTP and a molecule selected from the group consisting of an antibody, antibody fragment, and antibody-like binding molecule, wherein said molecule has a higher affinity for binding to a tumor than binding to other cells.

15. The use of claim 8, wherein the NTP is conjugated or linked or bound to a protein or other molecule, wherein the composition is cleaved at or near the site (s) of the tumor or other unwanted cells by a tumor-or site-specific enzyme or protease or by an antibody conjugate that targets tumor and so releases the NTP.

16. The use of claim 8, wherein the NTP is conjugated or linked or bound to a protein or other molecule, wherein the composition releases the NTP upon exposure of the tissue to be treated to light (as in laser therapies or other photo-dynamic or photo-activated therapy), other forms of electro-magnetic radiation such as infra-red radiation, ultraviolet radiation, x-ray or gamma ray radiation, localized heat, alpha or beta radiation, ultrasonic emissions, or other sources of localized energy.

17. The use of claim 8, wherein the NTP is to be employed in combination with other pharmaceutical compositions, such as cytokines, growth factors, antibiotics, apoptotisinducing agents, anti-inflammatories, and/or chemotherapeutic agents as is appropriate for the indication being treated.

18. The use of claim 8, where the NTP is to be employed in combination with dendrimers, fullerenes and other synthetic molecules, polymers and macromolecules wherin the NTP is conjugated with, attached to or enclosed in the molecule, polymer or macromolecule, either by itself or in conjunction with a molecule with a higher affinity for binding to a tumor than binding to other cells.

19. The use of claim 8, wherein the NTP is part of a single new cloned recombinant molecule consisting of NTP and a molecule selected from the group consisting of an antibody, antibody fragment, and antibody-like binding molecule, wherein said molecule has a higher affinity for binding to a tumor than binding to other cells.

20. Use of a therapeutically effective amount of an NTP gene for the manufacture of a composition for treating tumors.

## Patentansprüche

1. Anwendung einer therapeutisch effektiven Menge eines Neurofilamentproteins (NPT) zur Herstellung einer Zusammensetzung zur Behandlung von Tumoren.

2. Anwendung nach Anspruch 1, worin das NTP durch ein Verfahren verabreicht wird, das aus der Gruppe bestehend aus oral, subkutan, intradermal, intravenös, intramuskulär, intrathekal, intranasal, intratumoral, topisch und transdermal ausgewählt ist.

3. Anwendung nach irgendeinem der Ansprüche 1 oder 2, worin die Behandlung dem Patienten vor, während oder nach der Behandlung des Patienten verabreicht wird, ausgewählt aus der Gruppe von Behandlungen bestehend aus chirurgischer Exzision, Transplantation, "Grafting", Chemotherapie, Immuntherapie, Beimpfung, thermischer oder elektrischer Ablation, Kryotherapie, Lasertherapie, Phototherapie, Gentherapie und Strahlung.

4. Anwendung nach irgendeinem der Ansprüche 1 bis 3, worin der Tumor aus der Gruppe ausgewählt ist, bestehend aus einem gutartigen oder bösartigen Tumor eines Gewebes aus der Gruppe bestehend aus Lunge, Brust, Magen, Pankreas, Prostata, Blase, Knochen, Ovarium, Haut, Niere, Sinus, Dickdarm, Darm, Magen, Rektum, Speiseröhre, Herz, Milz, Speicheldrüse, Blut, Gehirn und dessen Abdeckungen, Rückenmark und dessen Abdeckungen, Muskel, Bindegewebe, Nebenniere, Parathyroid, Thyroid, Gebärmutter, Hoden, Hypophyse, Fortpflanzungsorganen, Leber, Gallenblase, Auge, Ohr, Nase, Hals, Mund und Lymphknoten und Lympfsystem ausgewählt ist.

5. Anwendung nach Anspruch 4, worin das Gewebe aus der Gruppe bestehend aus Haut, Auge, Ohr, Nase, Hals, Mund, Muskel, Bindegewebe, Haar und Brust ausgewählt ist.

6. Anwendung nach irgendeinem der Ansprüche 1 bis 5, worin das NTP an ein Molekül konjugiert, verbunden oder gebunden ist, das aus der Gruppe bestehend aus Antikörper, Antikörperfragment und einem antikörperähnlichen bindenden Molekül ausgewählt ist, wo das Molekül eine höhere Affinität zur Bindung an einen Tumor als Bindung an andere Zellen hat.

7. Anwendung nach irgendeinem der Ansprüche 1 bis 6, worin das NTP Teil eines einzelnen neuen geklonten rekombinanten Moleküls ist, bestehend aus NTP und einem Molekül, das aus der Gruppe bestehend aus Antikörper, Antikörperfragment und einem antikörperähnlichen bindenden Molekül ausgewählt ist, wo das Molekül eine höhere Affinität zur Bindung an einen Tumor als Bindung an andere Zellen hat.

8. Anwendung nach irgendeinem der Ansprüche 1 bis 7, worin das NTP aus der Gruppe bestehend aus AD7c-NTP (SEQ ID NO. 1), den Proteinen, die durch SEQ ID NO. 2 bis 9 identifiziert sind, neuralem Bauchspeicheldrüse-Filamentprotein und Bauchspeicheldrüse-Filamentprotein ausgewählt ist.

9. Anwendung nach Anspruch 8, worin das NTP durch ein Verfahren verabreicht wird, das aus der Gruppe bestehend aus oral, subkutan, intradermal, intravenös, intramuskulär, intrathekal, intraperitoneal, intracerebral (intraparenchymal), intracerebroventrikulär, intraokular, intraarteriell, intranasal, intratumoral, intraläsional, topisch und transdermal ausgewählt ist.

10. Anwendung nach Anspruch 8, worin die Behandlung dem Patienten vor, während oder nach der Behandlung des Patienten verabreicht wird, ausgewählt aus der Gruppe von Behandlungen bestehend aus chirurgischer Exzision, Transplantation, "Grafting", Chemotherapie, Immuntherapie, Beimpfung, thermischer oder elektrischer Ablation, Kryotherapie, Lasertherapie, Phototherapie, Gentherapie und Strahlung.

11. Anwendung nach Anspruch 8, worin der Tumor ein gutartiger oder bösartiger Tumor eines Gewebes ist, das aus der Gruppe bestehend aus Lunge, Brust, Magen, Pankreas, Prostata, Blase, Knochen, Ovarium, Haut, Niere, Sinus, Dickdarm, Darm, Magen, Rektum, Speiseröhre, Herz, Milz, Speicheldrüse, Blut, Gehirn und dessen Abdeckungen, Rückenmark und dessen Abdeckungen, Muskel, Bindegewebe, Nebenniere, Parathyroid, Thyroid, Gebärmutter, Hoden, Hypophyse, Fortpflanzungsorganen, Leber, Gallenblase, Auge, Ohr, Nase, Hals, Mandeln, Mund und Lymphknoten und Lympfsystem ausgewählt ist.

12. Anwendung nach Anspruch 11, worin das Gewebe aus der Gruppe bestehend aus Haut, Auge, Ohr, Nase, Hals, Mund, Muskel, Bindegewebe, Fettgewebe, Haar und Brust ausgewählt ist.

13. Anwendung nach Anspruch 8, worin das NTP an ein Molekül konjugiert, verbunden oder gebunden ist, das aus der Gruppe bestehend aus Antikörper, Antikörperfragment und einem antikörperähnlichen bindenden Molekül ausgewählt ist, wo das Molekül eine höhere Affinität zur Bindung an einen Tumor als Bindung an andere Zellen hat.

14. Anwendung nach Anspruch 8, worin das NTP Teil eines einzelnen neuen geklonten rekombinanten Moleküls ist, bestehend aus NTP und einem Molekül, das aus der Gruppe bestehend aus Antikörper, Antikörperfragment und einem antikörperähnlichen bindenden Molekül ausgewählt ist, wo das Molekül eine höhere Affinität zur Bindung an einen Tumor als Bindung an andere Zellen hat.

15. Anwendung nach Anspruch 8, worin das NTP an ein Protein oder ein anderes Molekül konjugiert oder verbunden oder gebunden ist, wo die Zusammensetzung an der Stelle/den Stellen oder in der Nähe der Stelle(n) des Tumors oder anderer unerwünschter Zellen durch ein tumor- oder stellenspezifisches Enzym oder eine tumor- oder stellenspezifische Protease oder durch ein Antikörperkonjugat, das Tumor abzielt, gespalten wird und so das NTP freigibt.

16. Anwendung nach Anspruch 8, worin das NTP an ein Protein oder ein anderes Molekül konjugiert oder verbunden oder gebunden ist, wo die Zusammensetzung das NTP nach Belichtung des zu behandelnden Gewebes mit Licht (wie bei Lasertherapien oder anderer photodynamischer oder photoaktivierter Therapie), anderen Formen von elektromagnetischer Strahlung, wie z.B. infraroter Strahlung, ultravioletter Strahlung, Röntgen- oder Gammastrahlung, lokaler Wärme, Alpha- oder Betastrahlung, Ultraschallemissionen oder anderen Quellen von lokaler Energie freigibt.

17. Anwendung nach Anspruch 8, worin das NTP in Kombination mit anderen pharmazeutischen Zusammensetzungen, wie z.B. Cytokinen, Wachstumsfaktoren, Antibiotika, apoptoseinduzierenden Mitteln, entzündungshemmenden Mitteln und/oder chemotherapeutischen Mitteln verwendet wird, die für die Indikation, die behandelt wird, angemessen sind.

18. Anwendung nach Anspruch 8, worin das NTP in Kombination mit Dendrimeren, Fullerene und anderen synthetischen Molekülen, Polymeren und Makromolekülen verwendet wird, wo das NTP mit dem Molekül, Polymer oder Makromolekül konjugiert ist, daran gebunden ist oder darin eingeschlossen ist, entweder alleine oder zusammen mit einem Molekül mit höherer Affinität zur Bindung an einen Tumor als Bindung an andere Zellen.

19. Anwendung nach Anspruch 8, worin das NTP Teil eines einzelnen neuen geklonten rekombinanten Moleküls ist, bestehend aus NTP und einem Molekül, das aus der Gruppe bestehend aus Antikörper, Antikörperfragment und einem antikörperähnlichen bindenden Molekül ausgewählt ist, wo das Molekül eine höhere Affinität zur Bindung an einen Tumor als Bindung an andere Zellen hat.

20. Anwendung einer therapeutisch effektiven Menge eines NPT-Gens zur Herstellung einer Zusammensetzung zur Behandlung von Tumoren.

## Revendications

1. Utilisation d'une quantité thérapeutiquement efficace d'une protéine de fibre nerveuse (NTP) pour la préparation d'une composition de traitement de tumeurs.

2. Utilisation selon la revendication 1, dans laquelle la NTP doit être administrée par une voie choisie dans le groupe constitué des voies orale, sous-cutanée, intradermale, intraveineuse, intramusculaire, intrathécale, intranasale, intratumorale, topique et transdermale.

3. Utilisation selon l'une quelconque des revendications 1 ou 2, dans laquelle ledit traitement doit être administré au patient avant, pendant ou après le traitement du patient choisi, dans le groupe de traitements constitué de l'excision chirurgicale, la transplantation, le pontage, la chimiothérapie, l'immunothérapie, la vaccination, l'ablation thermale ou électrique, la cryothérapie, la thérapie au laser, la photothérapie, la thérapie génique et la radiation.

4. Utilisation selon l'une quelconque des revendications 1 à 3, dans laquelle la tumeur est choisie dans le groupe constitué d'une tumeur bénigne ou maligne d'un tissu choisi dans le groupe constitué des poumons, les seins, l'estomac, le pancréas, la prostate, la vessie, les os, l'ovaire, la peau, les reins, le sinus, le colon, les intestins, l'estomac, le rectum, l'oesophage, le coeur, la rate, le glandes salivaires, le sang, le cerveau et ses hémisphères, la corde spinale et ses enveloppes, les muscles, le tissu conjonctif, les surrénales, la parathyroïde, la thyroïde, l'utérus, les testicules, l'hypophyse, les organes reproductifs, le foie, la vésicule biliaire, les yeux, les oreilles, le nez, la gorge, la bouche, les glandes lymphatiques et le système lymphatique.

5. Utilisation selon la revendication 4, dans laquelle le tissu est choisi dans le groupe constitué de la peau, les yeux, les oreilles, le nez, la gorge, la bouche, les muscles, le tissu conjonctif, les cheveux et les seins.

6. Utilisation selon l'une quelconque des revendications 1 à 5, dans laquelle la NTP est conjuguée, liée ou fixée à une molécule choisie dans le groupe constitué d'un anticorps, un fragment d'anticorps et une molécule de fixation similaire à un anticorps, dans laquelle ladite molécule à une affinité plus forte pour se fixer à une tumeur que pour se fixer à d'autres cellules.

7. Utilisation selon l'une quelconque des revendications 1 à 6, dans laquelle la NTP fait partie d'une molécule clonée recombinante solitaire nouvelle constituée d'une NTP et d'une molécule choisie dans le groupe constitué d'un anticorps, un fragment d'anticorps et une molécule de fixation similaire à un anticorps, dans laquelle ladite molécule à une affinité plus forte pour se fixer à une tumeur que pour se fixer à d'autres cellules.

8. Utilisation selon l'une quelconque des revendications 1 à 7, dans laquelle ladite NTP est choisie dans le groupe constitué de l'AD7c-NTP (SEQ ID NO. 1), les protéines identifiées par les SEQ ID NOs 2 à 9, la protéine neurale PTP (pancreatic thread protein) et la protéine PTP.

9. Utilisation selon la revendication 8, dans laquelle la NTP doit être administrée par une voie choisie dans le groupe constitué des voies orale, sous-cutanée, intradermale, intraveineuse, intramusculaire, intrathécale, intrapéritonéale, intracérébrale (intraparenchymale), intracérébroventriculaire, intraoculaire, intraartérielle, intranasale, intratumorale, intralésionelle, topique et transdermale.

10. Utilisation selon la revendication 8, dans laquelle ledit traitement doit être administré au patient avant, pendant ou après le traitement du patient, choisi dans le groupe de traitements constitué de l'excision chirurgicale, la transplantation, le pontage, la chimiothérapie, l'immunothérapie, la vaccination, l'ablation thermale ou électrique, la cryothérapie, la thérapie au laser, la photothérapie, la thérapie génique et la radiation.

11. Utilisation selon la revendication 8, dans laquelle la tumeur est une tumeur bénigne ou maligne d'un tissu choisi dans le groupe constitué des poumons, les seins, l'estomac, le pancréas, la prostate, la vessie, les os, l'ovaire, la peau, les reins, le sinus, le colon, les intestins, l'estomac, le rectum, l'oesophage, le coeur, la rate, les glandes salivaires, le sang, le cerveau et ses hémisphères, la corde spinale et ses enveloppes, les muscles, le tissu conjonctif, les surrénales, la parathyroïde, la thyroïde, l'utérus, les testicules, l'hypophyse, les organes reproductifs, le foie, la vésicule biliaire, les yeux, les oreilles, le nez, la gorge, les tonsilles, la bouche, les glandes lymphatiques et le système lymphatique.

12. Utilisation selon la revendication 11, dans laquelle le tissu est choisi dans le groupe constitué de la peau, les yeux, les oreilles, le nez, la gorge, la bouche, les muscles, le tissu conjonctif, le tissu adipeux, les cheveux et les seins.

13. Utilisation selon la revendication 8, dans laquelle la NTP est conjuguée, liée ou fixée à une molécule choisie dans le groupe constitué d'un anticorps, un fragment d'anticorps et une molécule de fixation similaire à un anticorps, dans laquelle ladite molécule à une affinité plus forte pour se fixer à une tumeur que pour se fixer à d'autres cellules.

14. Utilisation selon la revendication 8, dans laquelle la NTP fait partie d'une molécule clonée recombinante solitaire nouvelle constituée d'une NTP et d'une molécule choisie dans le groupe constitué d'un anticorps, un fragment d'anticorps, et une molécule de fixation similaire à un anticorps, dans laquelle ladite molécule à une affinité plus forte pour se fixer à une tumeur que pour se fixer à d'autres cellules.

15. Utilisation selon la revendication 8, dans laquelle la NTP est conjuguée ou liée ou fixée à une protéine ou une autre molécule, dans laquelle la composition est clivée dans ou près du/des site(s) de la tumeur ou d'autres cellules non désirées par un enzyme ou une protéase spécifique à la tumeur ou au site ou par un conjugué d'anticorps qui cible la tumeur et libère ainsi la NTP.

16. Utilisation selon la revendication 8, dans laquelle la NTP est conjuguée ou liée ou fixée à une protéine ou une autre molécule, dans laquelle la composition libère la NTP après une exposition du tissu à traiter à la lumière (comme dans les thérapies au laser ou d'autres thérapies photo-dynamique ou photo-activée), à d'autres formes de radiation électromagnétique, comme la radiation infrarouge, la radiation UV, la radiation aux rayons X ou rayons gamma, la chaleur localisée, la radiation alpha ou beta, les émissions d'ultrason ou d'autres sources d'énergie localisée.

17. Utilisation selon la revendication 8, dans laquelle la NTP doit être utilisée en combinaison avec d'autres compositions pharmaceutiques, comme les cytokines, les facteurs de croissance, les antibiotiques, les agents inducteur d'apoptose, les anti-inflammatoires et/ou les agents de chimiothérapie comme approprié pour l'indication étant traitée.

18. Utilisation selon la revendication 8, dans laquelle la NTP doit être employée en combinaison avec des dendrimères, des fullerènes et d'autres molécules synthétiques, des polymères et des macromolécules, où la NTP est conjuguée avec, attachée à ou enveloppée dans la molécule, le polymère ou la macromolécule, soit seule, soit en combinaison avec une molécule avec une affinité plus forte pour se fixer à une tumeur que pour se fixer à d'autres cellules.

19. Utilisation selon la revendication 8, dans laquelle la NTP fait partie d'une molécule clonée recombinante solitaire nouvelle constituée d'une NTP et d'une molécule choisie dans le groupe constitué d'un anticorps, un fragment d'anticorps, et une molécule de fixation similaire à un anticorps, dans laquelle ladite molécule à une affinité plus forte pour se fixer à une tumeur que pour se fixer à d'autres cellules.

20. Utilisation d'une quantité thérapeutiquement efficace d'un gène NTP pour la préparation d'une composition de traitement de tumeurs.
